(19) **Europäisches Patentamt / European Patent Office / Office européen des brevets**

(11) **EP 4 649 981 A1**

(12) **EUROPEAN PATENT APPLICATION**
published in accordance with Art. 153(4) EPC

(43) Date of publication:
**19.11.2025 Bulletin 2025/47**

(21) Application number: **23915380.2**

(22) Date of filing: **13.01.2023**

(51) International Patent Classification (IPC):
**A61M 16/00** (2006.01)  **A61B 5/087** (2006.01)

(52) Cooperative Patent Classification (CPC):
**A61B 5/087; A61M 16/00**

(86) International application number:
**PCT/CN2023/072158**

(87) International publication number:
**WO 2024/148611 (18.07.2024 Gazette 2024/29)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Shenzhen Mindray Bio-Medical Electronics Co., Ltd.**
**Shenzhen, Guangdong 518057 (CN)**

(72) Inventors:
• LIU, Jinglei
Shenzhen, Guangdong 518057 (CN)
• HUANG, Zhiwen
Shenzhen, Guangdong 518057 (CN)
• ZHOU, Xiaoyong
Shenzhen, Guangdong 518057 (CN)
• SONG, Haisong
Shenzhen, Guangdong 518057 (CN)

(74) Representative: **KIPA AB**
**Drottninggatan 11**
**252 21 Helsingborg (SE)**

(54) **MEDICAL DEVICE, VENTILATION DEVICE, AND VENTILATION REGULATION AND CONTROL METHOD**

(57) A ventilation device and a ventilation regulation and control method. The method comprises: step 1a, acquiring a monitored pressure and/or airway flow rate of a ventilation device during the process of mechanical ventilation for a patient; step 2a, determining parameter values of respiration driving parameters according to the monitored pressure and/or airway flow rate, wherein the respiration driving parameters are used for representing the respiration effort of the patient; step 3a, performing statistical analysis on the parameter values of the respiration driving parameters, so as to determine a first target value; step 4a, comparing the first target value with a preset first range; and step 5a, according to the comparison result, adjusting and/or giving a prompt to adjust a parameter value of a ventilation support parameter, wherein the ventilation support parameter is used for reflecting the condition of the pressure or flow rate, which is set by the ventilation device. By means of the medical device, ventilation device and ventilation regulation and control method, lung injury or diaphragm fatigue caused by hyperactive autonomous respiration can be reduced, such that a better ventilation effect is achieved.

Obtain a monitoring pressure and/or an airway flow rate during mechanical ventilation for a patient
— 1a

Determine a parameter value of a respiratory drive parameter according to the monitoring pressure and/or the airway flow rate
— 2a

Perform a statistical analysis on the parameter value of the respiratory drive parameter, so as to determine a first target value
— 3a

Compare the first target value with a preset first range
— 4a

Adjust and/or prompt to adjust a parameter value of a ventilation support parameter according to a comparison result
— 5a

**FIG. 4**

**Description**

TECHNICAL FIELD

The disclosure relates to a technical field of medical technology, and in particular, relates to a medical device, a ventilation device, and a ventilation regulation and control method.

BACKGROUND

Mechanical ventilation is one of most important life support methods to save lives of critically ill patients, which can help patients with respiratory failure to maintain normal oxygenation and $CO_2$ excretion. Currently, a main method of mechanical ventilation in clinical practice is positive pressure ventilation, that is, giving a patient a certain volume or pressure of ventilation support during inhalation, and maintaining a certain positive end-expiratory pressure during exhalation to prevent alveolar collapse.

Clinical studies have shown that while positive pressure ventilation helps maintaining gas exchange in patient, it also causes certain damage to a respiratory system of patient. For example, positive pressure ventilation leads to excessively high transpulmonary pressure, and causes respiratory muscle-related lung injury, which in turn leads to an increase in a mortality rate of critically ill patient. While long-term positive pressure ventilation leads to a disuse atrophy of a diaphragm of patient, which in turn leads to a prolonged machine-operation time of patient and a delayed machine-weaning time.

Currently, clinical lung protection strategies, such as low tidal volume ventilation, limited plateau pressure and drive pressure, are only effective when a patient has no spontaneous respiration or has weak spontaneous respiration. When a spontaneous respiration of the patient is too strong, even if conditions, such as small tidal volume and low drive pressure, are satisfied, a transpulmonary pressure of the patient is still very high, which means that there is still a high risk of lung injury. How to better prevent lung injury and protect the diaphragm during mechanical ventilation is one of issues that need to be solved or improved.

SUMMARY

According to a first aspect, an embodiment provides a ventilation regulation and control method, including:

obtaining a monitoring pressure and/or an airway flow rate of a ventilation device during mechanical ventilation for a patient;

determining a parameter value of a respiratory drive parameter according to the monitoring pressure and/or the airway flow rate, wherein the respiratory drive parameter is for characterizing a respiratory effort of the patient, and includes a respiratory muscle pressure, inspiratory work or a flow amount index; wherein the respiratory muscle pressure is calculated according to the monitoring pressure and/or the airway flow rate; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

performing a statistical analysis on the parameter value of the respiratory drive parameter, so as to determine a first target value;

comparing the first target value with a preset first range, and adjusting and/or prompting to adjust a parameter value of a ventilation support parameter according to a comparison result, wherein the ventilation support parameter is for reflecting a pressure or a flow amount condition which is set by the ventilation device.

According to a second aspect, an embodiment provides a ventilation regulation and control method, including:

obtaining a monitoring pressure and/or an airway flow rate of a ventilation device during mechanical ventilation for a patient;

determining a parameter value of a respiratory drive parameter according to the monitoring pressure and/or the airway flow rate, wherein the respiratory drive parameter is for characterizing a respiratory effort of the patient, and includes a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

performing a statistical analysis on the parameter value of the respiratory drive parameter, so as to determine a first target value;

comparing the first target value with a preset first range, and adjusting and/or prompting to adjust a medication which affects the respiratory effort according to a comparison result.

According to a third aspect, an embodiment provides a ventilation regulation and control method, including:

obtaining a monitoring pressure and/or an airway flow rate of a ventilation device during mechanical ventilation for a patient;

determining a parameter value of a respiratory drive parameter according to the monitoring pressure and/or the airway flow rate, wherein the respiratory drive parameter is for characterizing a respiratory effort of the patient, and includes a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

performing a statistical analysis on the parameter value of the respiratory drive parameter within a set time period, so as to determine a second target value, wherein the set time period includes N respiratory cycles, wherein N is an integer greater than or equal to 1;

according to a preset proportional relationship between a target value which corresponds to the respiratory drive parameter and a parameter value which corresponds to a ventilation support parameter, obtaining, according to the second target value, a reference value which corresponds to a ventilation support parameter of the ventilation device;

adjusting and/or prompting to adjust, according to the reference value, a parameter value of the ventilation support parameter of the ventilation device in a target respiratory cycle; wherein the target respiratory cycle is after the set time period.

According to a fourth aspect, an embodiment provides a ventilation device, including:

a ventilation apparatus, which is configured to generate a gas according to a preset setting which includes at least a ventilation support parameter;

a ventilation pipeline, which is connected with a respiratory system of a patient, so as to deliver the gas to the patient;

a sensor assembly, which includes at least a pressure sensor and a flow amount sensor, wherein the pressure sensor is configured to acquire a monitoring pressure of the ventilation device during mechanical ventilation of the patient, the flow amount sensor is configured to acquire an airway flow rate of the ventilation device during the mechanical ventilation of the patient;

a processor, which is in respective signal connection with the ventilation apparatus and the sensor assembly;

the processor is further configured to obtain the monitoring pressure acquired by the pressure sensor and/or the airway flow rate acquired by the flow amount sensor during the mechanical ventilation of the patient; and determine a parameter value of a respiratory drive parameter according to the monitoring pressure and/or the airway flow rate, wherein the respiratory drive parameter is for characterizing a respiratory effort of the patient, and includes a respiratory muscle pressure, inspiratory work or a flow amount index; wherein the respiratory muscle pressure is calculated according to the monitoring pressure and/or the airway flow rate; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

the processor is further configured to determine a first target value based on the parameter value of the respiratory drive parameter, compare the first target value with a preset first range, and adjust and/or prompt to adjust a parameter value of a ventilation support parameter according to a comparison result, wherein the ventilation support parameter is for reflecting a pressure or a flow amount condition which is set by the ventilation device.

According to a fifth aspect, an embodiment provides a ventilation device, including:

a ventilation apparatus, which is configured to generate a gas according to a set pressure or flow rate;

a ventilation pipeline, which is connected with a respiratory system of a patient, so as to deliver the gas to the patient;

a sensor assembly, which includes at least a pressure sensor and a flow amount sensor, wherein the pressure sensor is configured to acquire a monitoring pressure of the ventilation device during mechanical ventilation of the patient, the flow amount sensor is configured to acquire an airway flow rate of the ventilation device during the mechanical ventilation of the patient;

a processor, which is in respective signal connection with the ventilation apparatus and the sensor assembly, and further in communicative connection with an infusion pump;

the processor is further configured to obtain the monitoring pressure and/or the airway flow rate of the ventilation device during the mechanical ventilation of the patient; determine a parameter value of a respiratory drive parameter according to the monitoring pressure and/or the airway flow rate, wherein the respiratory drive parameter is for characterizing a respiratory effort of the patient, and includes a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

the processor is configured to determine a first target value based on the parameter value of the respiratory drive parameter, compare the first target value with a preset first range, and control the infusion pump to adjust and/or prompt to adjust a medication which affects the respiratory effort according to a comparison result.

According to a sixth aspect, an embodiment provides a ventilation device, including:

a ventilation apparatus, which is configured to generate a gas according to a preset setting which includes at least ventilation support parameter;

a ventilation pipeline, which is connected with a respiratory system of a patient, so as to deliver the gas to the patient;

a sensor assembly, which includes at least a pressure sensor and a flow amount sensor, wherein the pressure sensor is configured to acquire a monitoring pressure of the ventilation device during mechanical ventilation of the patient, the flow amount sensor is configured to acquire an airway flow rate of the ventilation device during mechanical ventilation of the patient;

a processor, which is in respective signal connection with the ventilation apparatus and the sensor assembly;

the processor is further configured to obtain the monitoring pressure acquired by the pressure sensor and/or the airway flow rate acquired by the flow amount sensor during the mechanical ventilation of the patient; and determine a parameter value of a respiratory drive parameter according to the monitoring pressure and/or the airway flow rate, wherein the respiratory drive parameter is for characterizing a respiratory effort of the patient, and includes a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

the processor is further configured to determine a second target value according to the parameter value of the respiratory drive parameter within a set time period; wherein the set time period includes N respiratory cycles, wherein N is an integer greater than or equal to 1;

according to a preset proportional relationship between a target value which corresponds to the respiratory drive parameter and a parameter value which corresponds to a ventilation support parameter, obtain, according to the second target value, a reference value which corresponds to a ventilation support parameter of the ventilation device; adjust and/or prompt to adjust, according to the reference value, a parameter value of the ventilation support parameter of the ventilation device in a target respiratory cycle; wherein the target respiratory cycle is after the set time period.

According to a seventh aspect, an embodiment provides a medical device, including:

a memory, which is configured to store a program; and
a processor, which is configured to implement the above method by executing the program stored in the memory.

According to an eighth aspect, an embodiment provides a computer-readable storage medium, including a program, wherein the program is capable of being executed by a processor to implement the methods of above-mentioned various aspects.

In the above-mentioned embodiments, during ventilating a patient, a parameter value of a respiratory drive parameter, which parameter is for characterizing a respiratory effort of the patient, is obtained according to a monitoring pressure and/or an airway flow rate of the patient. In some embodiments, a first target value is obtained according to the parameter value of the respiratory drive parameter, and then a parameter value of a ventilation support parameter is adjusted and/or prompted to be adjusted according to a comparison result between the first target value and a first range, or a medication which affects a respiratory effort is adjusted and/or prompted to be adjusted according to a comparison result between the first target value and a first range, so as to suppress a spontaneous respiration of the patient when the respiratory effort of the patient is too strong for preventing diaphragm fatigue and lung damage, and so as to enhance a respiratory drive of the patient when the respiratory effort of the patient is too weak for preventing diaphragm disuse atrophy.

In addition, a second target value can be obtained based on a parameter value of the respiratory drive parameter in one or several respiratory cycles before a target respiratory cycle, and a parameter value of the ventilation support parameter in the target respiratory cycle can be proportionally adjusted according to the second target value, so as to better adapt to a spontaneous respiration of the patient in the target respiratory cycle.

BRIEF DESCRIPTION OF THE DRAWINGS

FIG. 1 is a structural diagram of a monitor in an embodiment.
FIG. 2 is a block diagram of a ventilator according to an embodiment.
FIG. 3 is a block diagram of an anesthesia machine according to an embodiment.
FIG. 4 is a flow chart of a ventilation regulation and control method according to an embodiment.
FIG. 5 is a waveform diagram of an airway pressure, a respiratory muscle pressure, an airway flow rate and a ventilation volume according to an embodiment.
FIG. 6 is a waveform graph of an airway pressure, an esophageal pressure and an airway flow rate according to an embodiment.
FIG. 7 is a model diagram of a respiratory system of a human body according to an embodiment.
FIG. 8 is a flow chart of a ventilation regulation and control method according to another embodiment.
FIG. 9 is a flow chart of a ventilation regulation and control method according to yet another embodiment.

DESCRIPTION OF THE PREFERRED EMBODIMENTS

This disclosure will be further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the description are operable to distinguish different objects, rather than to describe a specific order. In addition, the terms "connection", and "linkage" mentioned in this disclosure include direct and indirect connection (linkage), unless otherwise specified.

The medical devices in this disclosure are used in various occasions. For example, the medical device of this disclosure may be a monitor or a monitoring module in some embodiments, may be a ventilation device such as a ventilator and an anesthesia machine in some embodiments, and may be another medical device with computing and processing capabilities in some embodiments, which are described below.

In some embodiments, the medical device may be a monitor.

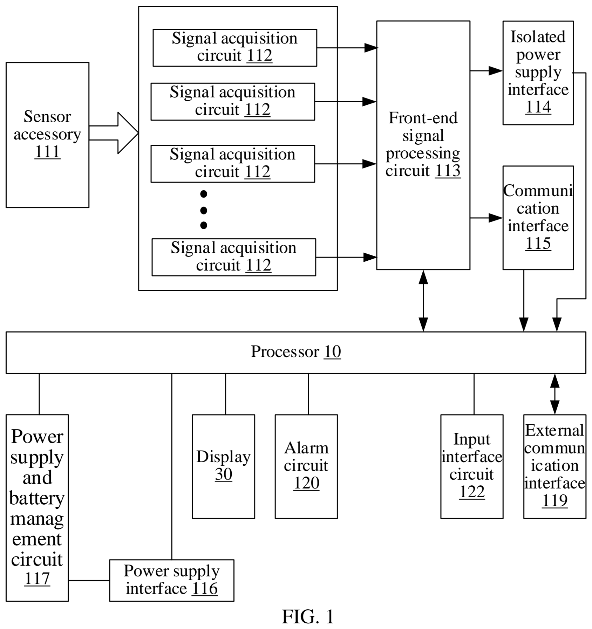

Referring FIG. 1, the monitoring device 100 may have an independent housing. A housing panel may have a sensor interface region in which multiple sensor interfaces are integrated for connecting with external physiological parameter sensor accessories 111. The housing panel further includes one or more of a small IXD display region, a display 30, an input interface circuit 122, and an alarm circuit 120 (such as an LED alarm region), etc. The medical device has an external communication interface 119 and a power supply interface 116 for communicating with and taking power from a main unit of a medical device, such as a patient monitor, a ventilator, an anesthesia machine. Airway pressure and airway flow rate are usually acquired by sensors in a ventilator/anesthesia machine, so that the medical device is capable of being in communicative connection with the ventilator/anesthesia machine to obtain the airway pressure and airway flow rate of the patient during ventilation. The medical device can be equipped with a carbon dioxide sensor or an electrical impedance sensor to acquire data, such as carbon dioxide concentration or chest electrical impedance of the patient during ventilation. Of course, the carbon dioxide sensor or the electrical impedance sensor can also be set in other devices (such as a ventilator), and the medical device obtains the carbon dioxide concentration or chest electrical impedance data by communicating with the other devices. The monitoring device can also support a build-out parameter module, can form a plug-in monitor main unit by means of inserting the parameter module, can be used as part of a monitor, or can be connected with the main unit via a cable, with the build-out parameter module being used as an external accessory of the monitoring device. The internal circuit of the medical device is placed in the housing, and may include a signal acquisition circuit 112 and a front-end signal processing circuit 113 corresponding to one or more physiological parameters. The signal acquisition circuit 112 can be selected from an electrocardiogram circuit, a respiratory circuit, a body temperature circuit, a blood oxygen circuit, a non-invasive blood pressure circuit, and an invasive blood pressure circuit, etc. These signal acquisition circuits 112 are in respective electrical connection with corresponding sensor interfaces and are used to be electrically connected with the sensor accessories 111 corresponding to different physiological parameters, with an output end thereof being coupled to the front-end signal processing circuit 113. A communication port of the front-end signal processing circuit 113 is coupled to the processor 10. The processor 10 is electrically connected with the external communication interface 119 and the power interface 116. The sensor accessories 111 and signal acquisition circuits 112 corresponding to various physiological parameters can adopt general circuits in the prior art. The front-end signal processing circuit 113 completes a sampling and analog-to-digital conversion of an output signal of the signal acquisition circuit 112, and outputs a control signal to control a measurement process of physiological signal. These parameters include but are not limited to: an electrocardiogram parameter, a respiration parameter, a body temperature parameter, a blood oxygen parameter, a non-invasive blood pressure parameter, and an invasive blood pressure parameter. The front-end signal processing circuit 113 can be implemented by a single-chip microcomputer or other semiconductor devices. The front-end signal processing circuit 113 can be powered by an isolated power supply. The sampled data is simply processed and packaged and then transmitted to the processor 10 through an isolated communication interface. For example, the front-end signal processing circuit 113 can be coupled to the processor 10 through an isolated power supply interface 114 and a communication interface 115. The reason that the front-end signal processing circuit 113 is powered by an isolated power source is that the DC/DC power source isolated by a transformer plays a role in isolating the patient from the power supply device, and the main purpose is: 1. isolating the patient, in which the application part is floated above the ground through the isolation transformer such that the leakage current of the patient is small enough; and 2. preventing the voltage or energy in the application of defibrillation or electric scalpel from affecting the boards and devices of the intermediate circuit such as the main control board (guaranteed by creepage distance and electrical clearance). Of course, the front-end signal processing circuit 113 can also be in direct connection with the processor 10 via a cable. The processor 10 is configured to complete a calculation of the physiological parameter, and send a calculation result and waveform of the parameter to a main unit (such as a main unit, a PC, a central station, etc., with a display 30) through the external communication interface 119. The processor 10 can be in direct communicative connection with the external communication interface 119 through a cable, and in direct connection with the power interface 116 through a cable for taking power from. The medical device can also include a power supply and battery management circuit 117, which draws power from the main unit through the power interface 116 and supplies the power to the processor 10 after processing, such as rectification and filtering; the power supply and battery management circuit 117 can also monitor, manage and protect the power obtained from the host through the power interface 116. The external communication interface 119 can be one or a combination of local region network interfaces composed of Ethernet, Token Ring, Token Bus, and the optical fibre distributed data interface (FDDI) as the backbone of these three networks, may also be one or a combination of wireless interfaces such as infrared, Bluetooth, WIFI, and WMTS communication, etc., or may also be one or a combination of wired data connection interfaces such as RS232 and USB etc. The external communication interface 119 may also be one or a combination of the wireless data transmission interface and the wired data transmission interface. The main unit can be any computer device, such as the main unit of a monitor, a computer, etc. The monitoring device can be formed by installing the matching software on the main unit. The main unit can also be a communication device, such as a mobile phone. The medical device transmits data to a mobile phone that supports Bluetooth communication through a Bluetooth interface to achieve remote data transmission. After the processor 10 completes the calculation of the physiological parameter, it can also determine whether the physiological parameter is abnormal. If yes, an alarm can be issued through the alarm circuit 120.

The above are some descriptions for a medical device as a monitor.

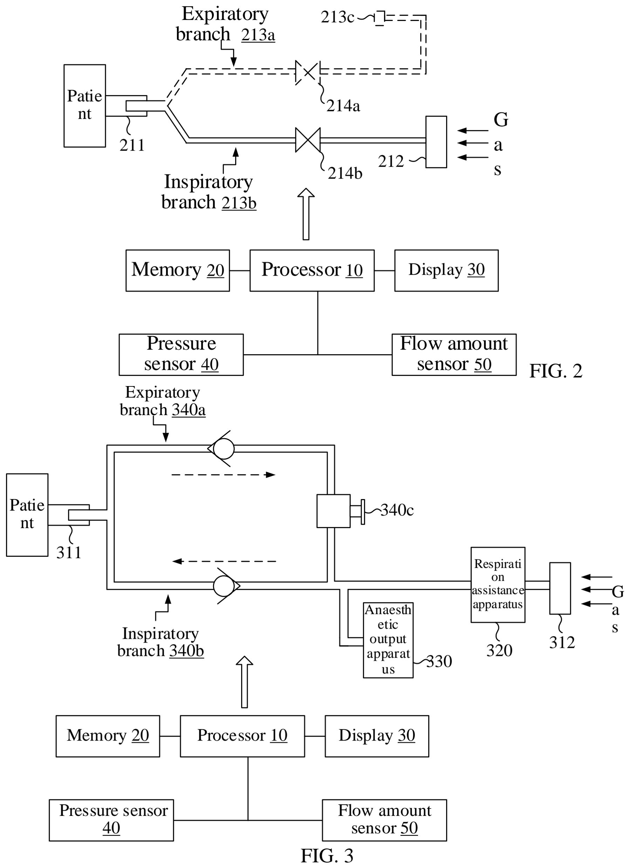

In some embodiments, the medical device is a ventilator, which is an artificial mechanical ventilation device used to assist or control a spontaneous respiration movement of a user, so as to achieve a function of gas exchange in lungs, reduce a body consumption, and facilitate a recovery of respiratory function. Please refer to FIG. 2, an invasive ventilator is shown, which includes a ventilation apparatus, a ventilation pipeline, a processor 10, a memory 20, a display 30 and a sensor assembly. The ventilation apparatus is configured to generate a gas according to a set ventilation support parameter, so as to ventilate the patient at a certain pressure or flow rate to provide respiratory support, wherein the ventilation support parameter includes but is not limited to a support pressure, an inspiratory pressure, a tidal volume, a ventilation volume per minute, etc. The ventilation apparatus includes at least a respiration assistance apparatus. The ventilation pipeline is connected with a respiratory system of the patient to deliver the gas to the patient. The ventilation pipeline includes a respiratory interface 211, a gas source interface 212 and a respiratory loop. The sensor assembly at least includes a pressure sensor 40 and a flow amount sensor 50.

The respiratory loop is selectively connected with the gas source interface 212 and a respiratory system of patient. In some embodiments, such as an invasive ventilator, the respiratory loop includes an expiratory branch 213a and an inspiratory branch 213b. The expiratory branch 213a is connected between the respiratory interface 211 and an exhaust port 213c to guide an exhaled gas of the patient to the exhaust port 213c. The exhaust port 213c can be connected with an external environment or to a dedicated gas recovery apparatus. In some embodiments, such as a non-invasive ventilator, the respiratory loop includes an inspiratory branch 213b but does not require an expiratory branch 213a. The gas source interface 212 is connected with a gas source (not shown in the drawing), which is configured to provide a gas, which can generally be oxygen, air, etc.. In some embodiments, the gas source can be a compressed gas cylinder or a central gas supply source, and the ventilator is supplied with gas through the gas source interface 212. Types of gas supplied include oxygen and air, etc. The gas source interface 212 may include conventional components, such as a pressure gauge, a pressure regulator, a flow meter, a pressure reducing valve, and an air-oxygen proportional control and protection apparatus, which are respectively used to control a flow amount of various gases (such as oxygen and air). Of course, for an electric ventilator, the gas source may also be a turbine, and the gas is outputted to the gas source interface 212 through the turbine. The inspiratory branch 213b is connected between the respiratory interface 211 and the gas source interface 212, and is configured to provide oxygen or air to the patient. For example, the gas inputted from the gas source interface 212 enters the inspiratory branch 213b and then enters the lungs of the patient through the respiratory interface 211. The respiratory interface 211 is configured to connect the patient to the respiratory loop, in addition to introducing the gas transmitted by the inspiratory branch 213b to the patient, for an invasive ventilator, the respiratory interface 211 can also introduce the gas exhaled by the patient into the exhaust port 213c through the expiratory branch 213a, and for a non-invasive ventilator, the respiratory interface 211 can also directly exhaust the gas exhaled by the patient. Depending on situations, the respiratory interface 211 can be a nasal cannula or a mask worn on the mouth and nose. The respiration assistance apparatus is connected with the gas source interface 212 and the respiratory loop to control the gas provided by the external gas source to be delivered to the patient through the respiratory loop. In an embodiment of an invasive ventilator, the respiration assistance apparatus may include an expiratory controller 214a and an inspiratory controller 214b, and in an embodiment of a non-invasive ventilator, the respiration assistance apparatus may include an inspiratory controller 214b. The expiratory controller 214a is arranged at the expiratory branch 213a, and is configured to open or close the expiratory branch 213a according to a control instruction, or to control a flow rate or pressure of the exhaled gas of the patient. In a specific implementation, the expiratory controller 214a may include one or more devices, such as an expiratory valve, a one-way valve, a flow amount controller, a PEEP valve, etc., that can control the flow amount or pressure. The inspiratory controller 214b is arranged at the inspiratory branch 213b, and is configured to open or close the inspiratory branch 213b according to a control instruction, or to control a flow rate or pressure of the outputted gas. In a specific implementation, the inspiratory controller 214b may include one or more devices, such as an inspiratory valve, a one-way valve or a flow amount controller, etc., that can control the flow amount or pressure.

The pressure sensor 40 is configured to acquire a monitoring pressure of the ventilator during mechanical ventilation of the patient, wherein the monitoring pressure includes but is not limited to an airway pressure, an esophageal pressure, etc. The flow amount sensor 50 is configured to acquire an airway flow rate of the ventilator during the mechanical ventilation of the patient.

The memory 20 can be used to store data or program(s), for example, to store data acquired by a sensor, data generated by the processor 10 through calculation, or an image frame generated by the processor 10, which image frame may be a 2D or 3D image, or the memory 20 may store a graphical user interface, one or more default image display settings, or programming instructions for the processor 10. The memory 20 may be a tangible and non-transitory computer-readable medium such as flash memory, RAM, ROM, EEPROM, etc.

The processor 10 is configured to execute instructions or programs, control the respiration assistance apparatus, the gas source interface 212 and/or various control valves and sensor components in the respiratory loop, or process received data to generate a required calculation or determination result or generate visual data or graphics, and output the visual data or graphics to the display 30 for display.

The above are some descriptions of the medical device as a ventilator. It should be noted that FIG. 2 above is only an example of a ventilator, and this is not intended to limit the ventilator to have only such a structure.

In some embodiments, the medical device may also be an anesthesia machine, which is mainly used to provide anesthetic gas, deliver the anesthetic gas to a respiratory system of the patient through a ventilator, and control an inhaled amount of the anesthetic gas. Please refer to the anesthesia machine shown in FIG. 3, which includes a ventilation apparatus, a ventilation pipeline, a processor 10, a memory 20, a display 30 and a sensor assembly. The ventilation device is configured to generate a gas according to a set pressure or a set flow amount to ventilate the patient to provide respiratory support. The ventilation device at least includes a respiratory assistance apparatus 320. The ventilation pipeline is connected with a respiratory system of a patient to deliver gas to the patient. The ventilation pipeline includes: a respiratory interface 311, a gas source interface 312 and a respiratory loop. The sensor assembly at least includes a pressure sensor 40 and a flow amount sensor 50.

The gas source interface 312 is connected with a gas source (not shown in the drawing), and the gas source is configured to provide a gas, which gas can usually be oxygen, nitrous oxide (laughing gas) or air. In some embodiments, the gas source may be a compressed gas cylinder or a central gas supply source, which supplies a gas to the anesthesia machine through the gas source interface 312, and the types of gas supplied include oxygen, nitrous oxide $N_2O$, air, etc. The gas source interface 312 may include conventional components, such as a pressure gauge, a pressure regulator, a flow meter, a pressure reducing valve, and an air-oxygen ratio proportional control and protection apparatus, which are respectively used to control a flow amount of various gases (such as oxygen, nitrous oxide, and air). The gas input from the gas source interface 312 enters the respiratory loop and forms a mixed gas with an original gas in the respiratory loop.

The respiration assistance apparatus 320 is configured to provide power for a non-spontaneous respiration of the patient and maintain airway patency. In some embodiments, the respiration assistance apparatus 320 is connected with the gas source interface 312 and the respiratory loop, so as to control the gas provided by the external gas source to be delivered to the patient through the respiratory loop. In some specific embodiments, the respiration assistance apparatus 320 mixes a fresh gas inputted from the gas source interface 312, a gas exhaled by the patient in the respiratory loop, and an anesthetic medication outputted from the anaesthetic output apparatus 330, and outputs the mixture to the respiratory interface 311 through the inspiratory branch 340b to drive the patient to inhale, and receives a gas exhaled by the patient through the expiratory branch 340a. In a specific embodiment, the respiratory assistance apparatus 320 generally includes a machine-controlled ventilation module, and a gas flow duct of the machine-controlled ventilation module is connected with the respiratory loop. During an anesthesia maintenance phase of an operation or when the patient has not recovered spontaneous respiration, a machine-controlled ventilation module is configured to provide the patient with respiratory power.

The anaesthetic output apparatus 330 is configured to provide an anaesthetic. Usually, the anaesthetic is mixed into a fresh air introduced by the gas source interface 312 in the form of gas and are delivered to the respiratory loop together. In a specific embodiment, the anaesthetic output apparatus 330 is implemented by an anaesthetic vaporizer. Anaesthetic is usually in a liquid form and stored in an anaesthetic vaporizer. Optionally, the anaesthetic vaporizer may include a heating device for heating the anaesthetic to volatilize it and generate anaesthetic vapor. The anaesthetic output apparatus 330 is connected with a pipeline of the gas source interface 312, and the anaesthetic vapor and the fresh air introduced by the gas source interface 312 are mixed and then transported together to the respiratory loop.

In some embodiments, the respiratory loop may include an inspiratory branch 340b, an expiratory branch 340a and a soda lime tank 340c. The inspiratory branch 340b and the expiratory branch 340a are connected with form a closed loop, and the soda lime tank 340c is arranged at a pipeline of the expiratory branch 340a. The mixed gas of fresh air introduced by the gas source interface 312 is inputted from an inlet of the inspiratory branch 340b and provided to the patient through the respiratory interface 311 arranged at an outlet of the inspiratory branch 340b. The respiratory interface 311 may be a mask, a nasal cannula or an endotracheal cannula. In a preferred embodiment, a one-way valve is provided at the inspiratory branch 340b, and the one-way valve is opened during an inspiratory phase and closed during an expiratory phase. The expiratory branch 340a is also provided with a one-way valve, which is closed during the inspiratory phase and opened during the expiratory phase. The inlet of the expiratory branch 340a is connected with the respiratory interface 311. When the patient exhales, the exhaled gas enters the soda lime tank 340c through the expiratory branch 340a, and carbon dioxide in the exhaled gas is filtered out by a substance in the soda lime tank 340c. The gas after being filtered out the carbon dioxide is recirculated into the inspiratory branch 340b.

The pressure sensor 40 is configured to acquire a monitoring pressure of the anesthesia machine during mechanical ventilation of the patient, wherein the monitoring pressure includes but is not limited to an airway pressure, an esophageal pressure, etc. The flow amount sensor 50 is configured to acquire an airway flow rate of the anesthesia machine during the mechanical ventilation of the patient.

The memory 20 can be used to store data or program(s), for example, to store data acquired by a sensor, data generated by the processor 10 through calculation, or an image frame generated by the processor 10, which image frame may be a 2D or 3D image, or the memory 20 may store a graphical user interface, one or more default image display settings, or programming instructions for the processor 10. The memory 20 may be a tangible and non-transitory computer-readable medium such as flash memory, RAM, ROM, EEPROM, etc.

The processor 10 is configured to execute instructions or programs, control the respiration assistance apparatus, the gas source interface 212 and/or various control valves and sensor components in the respiratory loop, or process received data to generate a required calculation or determination result or generate visual data or graphics, and output the visual data or graphics to the display 30 for display.

The above are some descriptions of the medical device as an anesthesia machine. It should be noted that FIG. 3 above is only an example of an anesthesia machine, and it is not intended to limit the anesthesia machine to have only such a structure.

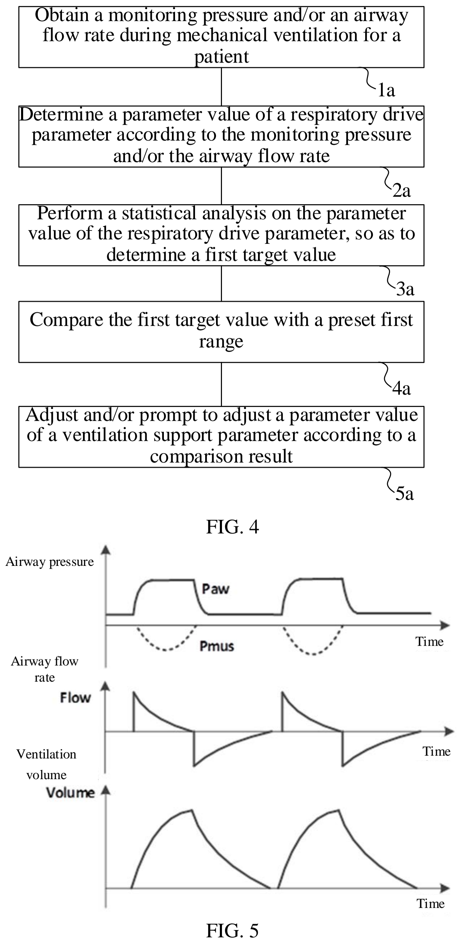

The following description takes the medical device as a ventilation device for example, and describes ventilation regulation and control methods of some embodiments of this disclosure with reference to FIG. 4.

Step 1a: obtain a monitoring pressure and/or an airway flow rate of a ventilation device during mechanical ventilation for a patient.

In this embodiment, the monitoring pressure includes but is not limited to an airway pressure, an esophageal pressure, etc. The pressure sensor 40 can be placed inside the machine (for example, arranged on a machine board), and which type of pressure, a measured pressure of the pressure sensor 40 belongs to, is determined by different positions of a sampling tube. For example, an airway pressure is measured, when the sampling tube is connected with a gas inlet or outlet end of the ventilator, or with a pipeline/mask. For example, an esophageal pressure is measured by placing a pressure measuring tube in an esophagus. The flow amount sensor 50 may also be arranged at a position which is connected with an airway of the patient, such as a respiratory interface or a respiratory loop. When the ventilation device ventilates the patient, the flow amount sensor 50 acquires an airway flow rate of the ventilation, that is, after acquiring the airway flow rate, an airway flow amount can be known according to an inner diameter of the pipeline, and a ventilation volume and the likes can be known according to a time.

Step 2a: determine a parameter value of a respiratory drive parameter according to the monitoring pressure and/or the airway flow rate. The parameter value of the respiratory drive parameter is capable of characterizing a strength of a respiratory effort of the patient. The larger the parameter value of the respiratory drive parameter, the stronger the respiratory effort of the patient. Conversely, the smaller the parameter value of the respiratory drive parameter, the weaker the respiratory effort of the patient.

In some embodiments, the respiratory drive parameter is a respiratory muscle pressure Pmus. Several methods for calculating the respiratory muscle pressure Pmus are first described below.

In some embodiments, when the monitoring pressure is an airway pressure of the patient, a respiratory muscle pressure can be calculated according to a respiratory mechanics equation group, as well as the airway pressure and an airway flow rate, wherein a respiratory system of a human body can be simplified to a first-order RC model, and the respiratory mechanics equation can be established according to the first-order RC model:

$$Paw\text{-}Pmus = Flow * R + Volume * E + PEEPtot .$$

Wherein R represents a viscous resistance of a respiratory system of the patient, E represents an elastic resistance of the respiratory system of the patient (which can also be represented by compliance C of the respiratory system of the patient); R and E are usually constants.

Pmus represents a respiratory muscle pressure; Paw represents an airway pressure, which can be directly measured by the pressure sensor 40 during mechanical ventilation; Flow represents an airway flow rate, which can be measured by a flow amount sensor 50; Volume represents a ventilation volume, which is obtained according to an integral value over time of the airway flow rate.

PEEPtot represents a total positive end-expiratory pressure. The total positive end-expiratory pressure PEEPtot is usually composed of two parts: exogenous positive end-expiratory pressure PEEPe and endogenous positive end-expiratory pressure PEEPi. As the name suggests, exogenous positive end-expiratory pressure (PEEPe) is a positive pressure in a respiratory tract of the patient at an expiratory end caused by external reason(s). It is usually generated when the ventilation device ventilates the patient. The user can adjust a size of the exogenous positive end-expiratory pressure (PEEPe) by setting the ventilation device. The endogenous positive end-expiratory pressure (PEEPi) is a positive pressure in the respiratory tract of the patient at the expiratory end caused by internal reason(s), such as a lung condition of the patient. The exogenous positive end-expiratory pressure PEEPe and the endogenous positive end-expiratory pressure PEEPi are finally added to form the total positive end-expiratory pressure PEEPtot. That is to say, PEEPtot is equal to the sum of the exogenous positive end-expiratory pressure PEEPe (exogenous PEEP, i.e. PEEP monitored by an airway pressure) and the endogenous positive end-expiratory pressure PEEPi (endogenous PEEP).

Some variables of the respiratory mechanics equation are shown in FIG. 5. The respiratory muscle pressure can be obtained by solving the respiratory mechanics equations to obtain each variable. Specifically, in the respiratory mechanics equation, PEEPtot, Pmus, R, and E are unknown quantities, and the others are known quantities. During an inhalation of the patient, Pmus changes with time. Then, by measuring a series of Paw, Flow, and Volume, multiple equations can be obtained:

$$\text{Paw}（1）-\text{Pmus}（1）=\text{Flow}（1）*\text{R}+\text{Volume}（1）*\text{E}+\text{PEEPtot};$$

$$\text{Paw}（2）-\text{Pmus}（2）=\text{Flow}（2）*\text{R}+\text{Volume}（2）*\text{E}+\text{PEEPtot};$$

$$...$$

$$\text{Paw}（n）-\text{Pmus}（n）=\text{Flow}（n）*\text{R}+\text{Volume}（n）*\text{E}+\text{PEEPtot}.$$

In other words, the processor 10 substitutes multiple groups of airway pressures Paw, airway flow rates Flow and ventilation volumes Volume, which groups are at different time points, as known quantities into a respiratory mechanics equation group, and obtains an equation group in which the unknown quantities are the pressure Pmus generated by the respiratory muscle and the total positive end-expiratory pressure PEEPtot. Taking n groups of Paw, Flow and Volume data as an example, n equations can be obtained, but there are n+3 unknowns. Therefore, in order to make the equation group solvable, this disclosure creatively simplifies Pmus. For example, according to a physiological characteristic of spontaneous respiration, Pmus is simplified to a known function (curve). In other words, in the respiratory mechanics equation, the respiratory muscle pressure is an unknown quantity that has a preset functional relationship with time. Although the respiratory muscle pressure is an unknown quantity, it also has its own rules (having a preset functional relationship with time). The preset functional relationship may include a combination of one or more of: an exponential function, a trigonometric function, a piecewise function and a polynomial function. According to the physiological characteristic of spontaneous respiration, as shown in FIG. 5, an absolute value of Pmus changes in a trend from small to large, and then from large to small. Therefore, we can select an exponential function to simulate a section from small to large, and then select another exponential function to simulate a section from large to small. Of course, these two exponential functions actually constitute a piecewise function. Similarly, a linear function can also be selected to simulate the section from small to large, and then another linear function can also be selected to simulate the section from large to small. Similarly, these two linear functions actually constitute a piecewise function. Similarly, a trigonometric function, a multi-order polynomial function, etc., can also be selected to simulate. The number of unknown variables will vary depending on the specific functional relationship selected. For example, a sine function (such as $\sin(b_0+b_1t)$), a cosine function, a quadratic polynomial function (such as $a_1t+a_2t^2$) can be used to simulate Pmus. In this embodiment, a parabola (quadratic polynomial function) is configured to simplify Pmus as an example, and the above equations can be simplified as follows:

$$Y=[\text{Paw}(1),\text{Paw}(2),\ ...,\ \text{Paw}(n)]^T$$

$$F=[\text{Flow}(1),\text{Flow}(2),\ ...,\ \text{Flow}(n)]^T$$

$$V=[\text{Volume}(1),\text{Volume}(2),\ ...,\ \text{Volume}(n)]^T$$

$$\text{Let: } X=[F,V,1,t,t^2],\ A=[R,E,\text{PEEPtot},a_1,a_2]^T;$$

then, the above equation group can be expressed by matrix Y=X*A, and the respiratory muscle pressure Pmus can be obtained by solving the above equation group, that is, performing matrix solving to obtain the respiratory muscle pressure Pmus. Specifically, a number of data sampling points of a general inspiratory process (a number of groups of Paw, Flow and Volume data) is much greater than 5, so the equation group is an overdetermined equation group, that is, the number of n satisfies the requirement to solve n+3 overdetermined equations. If R and E are known, the requirement to solve n+1 overdetermined equations is satisfied. The optimal solution of the overdetermined equation group can be solved by the following process:

Y=X*A, then $X^T*Y=X^T*X*A$; wherein $X^T*X$ is a 5*5 matrix, which can be inverted, so as to obtain $A=(X^T*X)^{-1}X^T*Y$, and further obtain Pmus after a matrix solving.

Furthermore, in order to improve an accuracy and effectiveness of the solution, one or more constraints may be added in the process of solving the equation group, including but not limited to: setting the respective respiratory muscle pressure Pmus at a start moment and an end moment of inhalation to 0; setting the endogenous positive end-expiratory pressure PEEPi to be greater than or equal to 0; for a patient without spontaneous respiration, setting the respiratory muscle pressure Pmus to 0; limiting the viscous resistance R of the respiratory system of the patient and the elastic resistance E of the respiratory system of the patient to a preset reasonable range, which range may be set according to actual conditions, clinical experience, etc. One or more of these constraints can be selected as constraints when solving the equation group, so as to obtain the respiratory muscle pressure Pmus more accurately and reliably.

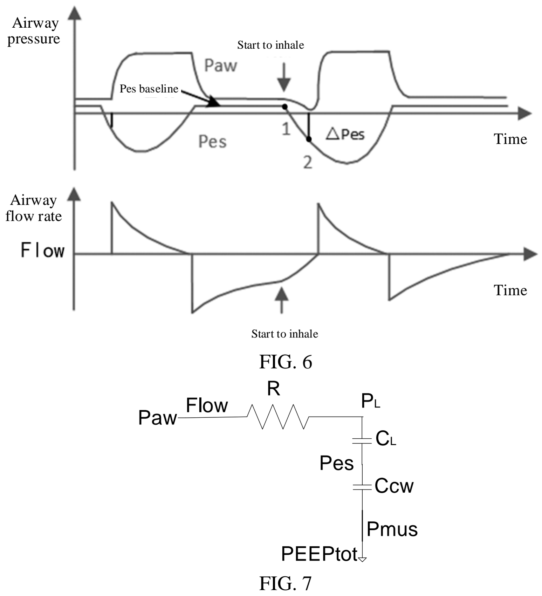

In some embodiments, when the monitoring pressure is an esophageal pressure of the patient, a chest wall compliance Ccw of the patient is calculated based on the esophageal pressure and the airway flow rate, wherein the chest wall compliance Ccw=△V/dPES, wherein △V represents a change in a lung capacity, which can be reflected by the airway flow rate, dPES represents a change in the esophageal pressure as the capacity changes, including a waveform change of esophageal pressure as shown in FIG. 6. After the chest wall compliance is obtained, the respiratory muscle pressure is calculated based on the airway flow rate, the esophageal pressure and the chest wall compliance according to a human respiratory system model. FIG. 7 is a schematic diagram of a human respiratory system model, wherein Paw is an airway pressure, Flow is an airway flow rate, R is a viscous resistance of a respiratory system of the patient, PL is an intrapulmonary pressure, CL is a lung compliance, Ccw is a chest wall compliance, Pes is an esophageal pressure, which is used clinically to approximate an intrathoracic pressure, and PEEPtot is a total positive end-expiratory pressure. According to the respiratory system model, the respiratory muscle pressure can be calculated through the esophageal pressure and the chest wall compliance, specifically: Pmus = Pes-Volume/Ccw.

In some embodiments, when the monitoring pressures are an esophageal pressure and an intragastric pressure of the patient, a transdiaphragmatic pressure of the patient is calculated based on the esophageal pressure and the intragastric pressure, wherein the transdiaphragmatic pressure is used as the respiratory muscle pressure, wherein transdiaphragmatic pressure = pleural pressure - intra-abdominal pressure. The transdiaphragmatic pressure can be obtained by approximately replacing the pleural pressure with the esophageal pressure and approximately replacing the intra-abdominal pressure with the intragastric pressure.

In some embodiments, when the monitoring pressure is an esophageal pressure of the patient, the esophageal pressure can also be directly used as the respiratory muscle pressure.

In some embodiments, the respiratory drive parameter is inspiratory work, which is obtained by an integral value over time of a product of the respiratory muscle pressure and the airway flow rate. After the respiratory muscle pressure is calculated by the method in any of the above embodiments, the integral value over time of the product of the respiratory muscle pressure and the airway flow rate is calculated to obtain the inspiratory work.

In some embodiments, the respiratory drive parameter is a pressure area of respiratory muscle. The pressure area of respiratory muscle is obtained based on an integral value over time of the respiratory muscle pressure. After the respiratory muscle pressure is calculated by adopting the method in any of the above embodiments, the integral value over time of the respiratory muscle pressure is calculated to obtain the pressure area of respiratory muscle. In FIG. 5, an area enclosed by a curve of the respiratory muscle pressure and a time axis in each respiratory cycle is the pressure area of respiratory muscle corresponding to said respiratory cycle.

In some embodiments, the respiratory drive parameter is a flow amount index. The flow amount index is for characterizing a rate for a change within a descending process of an inspiratory flow amount curve, which curve is obtained from the airway flow rate. Specifically, the flow amount index corresponding to one respiratory cycle can be calculated according to the following formula:

$$F = a + b\Delta t^{c}.$$

Wherein, F represents an airway flow rate in an inspiratory phase of a respiratory cycle, a represents a peak value of the airway flow rate in the inspiratory phase of the respiratory cycle, b represents a reduction rate of flow amount which rate corresponds to a selected time period $\Delta t$ in the inspiratory phase of the respiratory cycle, and c represents a flow amount index. For example, the selected time period can be a time from the peak value to one-quarter of the peak value of the airway flow rate in the inspiratory phase. It can be seen from the formula that the flow amount index can be obtained after determining the selected time period. In this embodiment, each respiratory cycle only uses one selected time period, so that each respiratory cycle corresponds to one flow amount index.

In some embodiments, after obtaining the parameter value of the respiratory drive parameter, the parameter value of the respiratory drive parameter can also be displayed. For example, when the medical device is a monitor, the parameter value of the respiratory drive parameter can be displayed, on the display 30 of the monitor, together with a parameter value of a physiological parameter of the patient. When the medical device is a ventilator or an anesthesia machine, the parameter value of the respiratory drive parameter can be displayed, on the display 30 of the ventilator or anesthesia machine, together with a ventilation support parameter.

Step 3a: perform a statistical analysis on the parameter value of the respiratory drive parameter, so as to determine a first target value.

It is explained above that the respiratory drive parameter includes a respiratory muscle pressure, inspiratory work, a pressure area of respiratory muscle or a flow amount index.

In some embodiments, if the respiratory drive parameter is a respiratory muscle pressure, the respiratory muscle pressure within a first set time period is obtained, and an associated value of any one of a maximum value, an average value and a median value of the respiratory muscle pressure within the first set time period is used as the first target value. In some embodiments, the associated value refers to a maximum value, an average value or a median value of the respiratory muscle pressure itself. For example, a maximum value of the respiratory muscle pressure within a first set time period is used as the first target value, or an average value of the respiratory muscle pressure within a first set time period is used as the first target value. In other embodiments, the associated value refers to a value obtained by performing a certain calculation based on a maximum value, an average value or a median value of the respiratory muscle pressure. For example, after obtaining a maximum value of the respiratory muscle pressure, the maximum value is corrected by using a preset correction coefficient to obtain the first target value. For another example, after obtaining a maximum value of the respiratory muscle pressure, a preset value is added or subtracted to obtain the first target value. In some embodiments, the first set time period is a time period of at least one respiratory cycle. As can be seen from FIG. 5, the respiratory muscle pressure is a variable that can be calculated in real time as it changes over time.

In some embodiments, if the respiratory drive parameter is inspiratory work, the first target value is determined according to the inspiratory work which is obtained from an integral value over a second set time period. In some embodiments, the second set time period is a time period of at least one respiratory cycle. In other embodiments, N inspiratory work which corresponds to N respiratory cycles can also be obtained, wherein N is an integer greater than or equal to 1, and each respiratory cycle corresponds to one inspiratory work. Then, an associated value of any one of a maximum value, an average value and a median value of the N inspiratory work is used as the first target value. In some embodiments, the associated value refers to a maximum value, an average value or a median value of the N inspiratory work itself. For example, a maximum value of the N inspiratory work is used as the first target value. In other embodiments, the associated value refers to a value obtained by performing a certain calculation based on a maximum value, an average value or a median value of N inspiratory work. For example, after obtaining an average value of N inspiratory work, the average value is corrected by using a preset correction coefficient to obtain the first target value. For another example, after obtaining an average value of N inspiratory work, a preset value is added or subtracted to obtain the first target value.

In some embodiments, if the respiratory drive parameter is a pressure area of respiratory muscle, the first target value is determined according to the pressure area of respiratory muscle which is obtained from an integral value over a third set time period of the respiratory muscle pressure. In some embodiments, the third set time period is a time period of at least one respiratory cycle. In other embodiments, N pressure areas of respiratory muscle which correspond to N respiratory cycles can also be obtained, wherein N is an integer greater than or equal to 1, and each respiratory cycle corresponds to one pressure area of respiratory muscle. For example, in FIG. 5, N is 2, and there are two pressure areas of respiratory muscle. Then, an associated value of any one of a maximum value, an average value and a median value of the N pressure areas of respiratory muscle is used as the first target value. In some embodiments, the associated value refers to a maximum value, an average value or a median value of the N pressure areas of respiratory muscle itself. For example, a maximum value of the N pressure areas of respiratory muscle is used as the first target value. In other embodiments, the associated value refers to a value obtained by performing a certain calculation based on a maximum value, an average value or a median value of N pressure areas of respiratory muscle. For example, after obtaining an average value of N pressure areas of respiratory muscle, the average value is corrected by using a preset correction coefficient to obtain the first target value. For another example, after obtaining an average value of N pressure areas of respiratory muscle, a preset value is added or subtracted to obtain the first target value.

In some embodiments, if the respiratory drive parameter is a flow amount index, N flow amount indices which correspond to N respiratory cycles are calculated, wherein N is an integer greater than or equal to 1. Then, an associated value of any one of a maximum value, an average value and a median value among the N flow amount indexes is used as the first target value. In some embodiments, the associated value refers to a maximum value, an average value or a median value of the N flow amount indexes itself. For example, a maximum value of the N flow amount indexes is used as the first target value. In other embodiments, the associated value refers to a value obtained by performing a certain calculation based on a maximum value, an average value or a median value of N flow amount indices. For example, after obtaining an average value of N flow amount indices, the average value is corrected by using a preset correction coefficient to obtain the first target value. For another example, after obtaining an average value of N flow amount indices, a preset value is added or subtracted to obtain the first target value.

Step 4a: compare the first target value with a preset first range.

Step 5a: adjust and/or prompt to adjust a parameter value of a ventilation support parameter according to a comparison result.

The respiratory drive parameter is capable of characterizing the respiratory effort of the patient, and the first target value obtained by the parameter value of the respiratory drive parameter can more accurately reflect the strength of a spontaneous respiration of the patient.

If the first target value is greater than an upper limit of the first range, it means that a current spontaneous respiration of the patient is too strong, so that the parameter value of the ventilation support parameter is increased and/or prompted to be increased to reduce an intensity of a spontaneous respiration of the patient and prevent diaphragm fatigue.

The method of increasing the parameter value of the ventilation support parameter may be that the ventilation device changes the parameter value of the ventilation support parameter according to a set rule. In some embodiments, the parameter value of the ventilation support parameter may be increased with a first adjustment step corresponding to the ventilation support parameter. For example, if the ventilation support parameter is a support pressure, a preset pressure value is increased based on a current support pressure. For another example, if the ventilation support parameter is a tidal volume, a preset tidal volume is increased based on a current tidal volume. Prompting to increase the parameter value of the ventilation support parameter may be displaying prompting information on the display 30, or prompting the user to increase the parameter value of the ventilation support parameter by sound, light, etc.

If the first target value is less than an lower limit of the first range, it means that a current spontaneous respiration of the patient is very weak, so that the parameter value of the ventilation support parameter is decreased and/or prompted to be decreased to enhance a respiratory drive of the patient and prevent diaphragmatic disuse atrophy. In some embodiments, the method for decreasing the parameter value of the ventilation support parameter is similar to that for increasing the parameter value of the ventilation support parameter, and the parameter value of the ventilation support parameter is also decreased by a second adjustment step corresponding to the ventilation support parameter. The first adjustment step and the second adjustment step may be the same or different. In summary, after obtaining the first target value, the first target value is compared with the first range. If the first target value is within the first range, it means that a spontaneous respiration of the patient is within an appropriate range. If the first target value is not within the first range, whether to decrease or increase the ventilation support parameters is determined according to whether the first target value is lower than or higher than the first range, and the adjustment process is a step-by-step adjustment.

In some embodiments, while adjusting and/or prompting to adjust the parameter value of the ventilation support parameter, the parameter value of the ventilation support parameter can also be compared with a preset support parameter range in real time or periodically. When the parameter value of the ventilation support parameter reaches a limit value of the support parameter range, stop adjusting or prompting to adjust the parameter value of the ventilation support parameter. The support parameter range can be set by the user based on clinical experience to avoid excessive larger or small ventilation support parameter from causing harm to the patient body. For example, if the first target value is greater than the upper limit of the first range, the parameter value of the ventilation support parameter begins to increase. When the parameter value of the ventilation support parameter reaches an upper limit of the support parameter range, the parameter value of the ventilation support parameter is no longer increased, making the adjustment of the ventilation support parameter safer.

The currently implemented PAV (Proportional Assist Ventilation) mode is that: determining a ventilation pressure at a next moment according to a preset ratio based on an acquired real-time parameter value which represents a respiratory effort, and controlling the ventilation device according to the determined ventilation pressure at the next moment; specifically, within one respiratory cycle, the greater the parameter value of the respiratory effort at the i-th moment, the greater the support pressure given by the ventilator at the i+1 moment. Compared with the existing PAV mode, the embodiment described in FIG. 4 above, is as follows. On the one hand, the first range is set by the user, and when the user does not adjust the first range, the first target value obtained through the statistical analysis is compared with a currently set first range. In this way, the user can set the first range by operating in the medical device, while the PAV mode does not require the first range to be set in advance. On the other hand, the PAV mode directly performs a proportional adjustment by acquiring a parameter value of the respiratory effort in real time, while this disclosure performs the comparison step only after obtaining the first target value through a statistical analysis of the parameter value of the respiratory drive parameter. Since the ventilation pressure adjusted by the real-time proportional adjustment method has a large fluctuation, it affects the stable regulation of the patient. This disclosure does not adopt a real-time regulation of the ventilation support parameter, which reduces a calculation complexity while achieving a stable regulation of the patient.

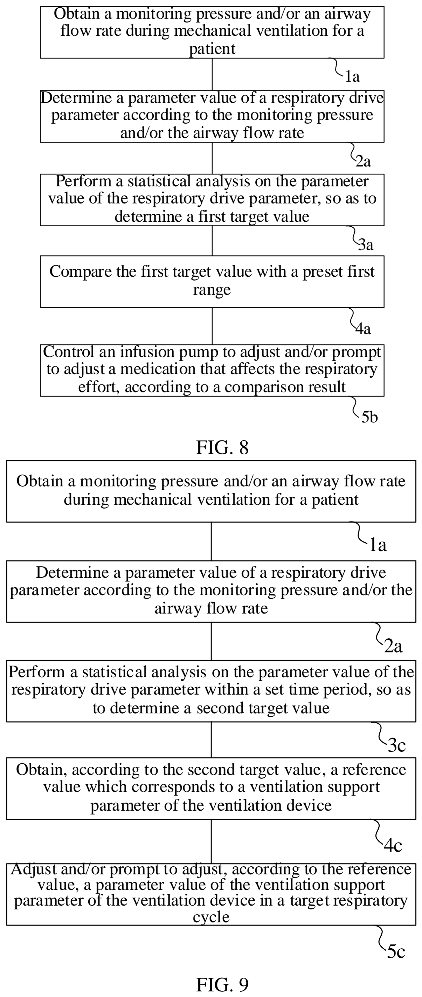

In some embodiments, the processor 10 is also in communicative connection with an infusion pump. The infusion pump is configured to deliver a selected medication into the patient body, and the ventilation device can transmit a control instruction to control an operation of the infusion pump, such as controlling what medication the infusion pump delivers to the patient, or controlling an infusion rate of the medication. As shown in FIG. 8, after step 4a, a following step is further included:

Step 5b: control an infusion pump to adjust and/or prompt to adjust a medication that affects the respiratory effort, according to a comparison result.

Specifically, if the first target value is greater than an upper limit of the first range, when the ventilation device is in communicative connection with the infusion pump, the ventilation device can directly control the infusion pump to increase a medication that affects the respiratory effort, such as a muscle relaxant, a sedative, etc. In some embodiments, increasing a medication can have two meanings. The first is that if the patient was not originally using a medication that affects the respiratory effort, then a medication that affects the respiratory effort starts to be supplied to the patient. The other meaning is that if the patient is currently using a medication that affects the respiratory effort, then an amount and/or a frequency of use of the medication is increased. In addition to increasing the medication, if the first target value is greater than the upper limit of the first range, the ventilation device may also prompt the user to increase the medication that affects the respiratory effort, for example, by displaying corresponding prompting information on the display 30 or transmitting corresponding prompting information to the infusion pump. It will be appreciated that a prompt to increase the medication may be issued regardless of whether the ventilation device is in communication with the infusion pump.

In some embodiments, when increasing and/or prompting to increase a medication, a first difference between the first target value and an upper limit of the first range is obtained, first medication administration data is determined according to the first difference, and the medication is increased and/or prompted to be increased by using the first medication administration data. In some embodiments, the first medication administration data includes a first medication administration dosage, and the first medication administration dosage is greater than a medication administration dosage before adjusting. In this case, increasing and/or prompting to increase the medication based on the first medication administration data means administering the medication at the first medication administration dosage and/or prompting to administer the medication at the first medication administration dosage.

In some embodiments, the first medication administration data includes a first medication administration frequency of a set dosage, and if the first medication administration frequency is greater than a medication administration frequency of the set dosage before adjusting, then increasing the medication and/or prompting to increase the medication according to the first medication administration data, means administering the medication at the first medication administration frequency and/or prompting to administer the medication at the first medication administration frequency, while maintaining the set dosage. It can be seen from the above description that increasing medication administration can be achieved by at least one of: increasing a dosage of medication administration and increasing a frequency of medication administration. In some embodiments, the larger the first difference is, the larger the first medication administration dosage is, and/or the higher the first medication administration frequency is.

If the first target value is less than an lower limit of the first range, when the ventilation device is in communicative connection with the infusion pump, the ventilation device can directly control the infusion pump to decrease a medication that affects the respiratory effort. In some embodiments, decreasing a medication can have two meanings. The first is that if the patient is originally using a medication that affects the respiratory effort, the infusion pump is controlled to no longer output the medication. The other meaning is that the patient is currently using a medication that affects the respiratory effort, then an amount and/or a frequency of use of the medication is decreased. In addition to decreasing the medication, if the first target value is less than the lower limit of the first range, the ventilation device may also prompt the user to reduce the medication that affects the respiratory effort, for example, by displaying a corresponding prompting information on the display 30 . It will be appreciated that a prompt to decrease the medication may be issued regardless of whether the ventilation device is in communication with the infusion pump.

In some embodiments, when decreasing and/or prompting to decrease a medication that affects the respiratory effort, a second difference between the first target value and the lower limit of the first range is obtained, second medication administration data is determined according to the second difference, and the medication is decreased and/or prompted to be decreased by using the second medication administration data. In some embodiments, the second medication administration data includes a second medication administration dosage, and the second medication administration dosage is less than a medication administration dosage before adjusting. In this case, decreasing and/or prompting to decrease the medication based on the second medication administration data means administering the medication at the second medication administration dosage and/or prompting to administer the medication at the second medication administration dosage.

In some embodiments, the second medication administration data includes a second medication administration frequency of a set dosage, and if the second medication administration frequency is less than a medication administration frequency of the set dosage before adjusting, then decreasing the medication and/or prompting to increase the medication according to the second medication administration data, means administering the medication at the second medication administration frequency and/or prompting to administer the medication at the second medication administration frequency, while maintaining the set dosage. It can be seen from the above description that decreasing administration can be achieved by at least one of: decreasing a dosage of medication administration and decreasing a frequency of medication administration. In some embodiments, the larger the second difference is, the fewer the second medication administration dosage is, and/or the lower the second medication administration frequency is.

In some embodiments, as shown in FIG. 9, another ventilation regulation and control method is provided, which includes after step 2a:

step 3c: perform a statistical analysis on the parameter value of the respiratory drive parameter within a set time period, so as to determine a second target value, wherein the set time period includes N respiratory cycles, wherein N is an integer greater than or equal to 1. That is to say, in this embodiment, the second target value is determined according to the parameter value of the respiratory drive parameter in at least one respiratory cycle.

Specifically, the respiratory drive parameter include a respiratory muscle pressure, a pressure area of respiratory muscle, inspiratory work, or a flow amount index.

In some embodiments, if the respiratory drive parameter is a respiratory muscle pressure, the respiratory muscle pressure within a set time period is obtained, and an associated value of any one of a maximum value, an average value and a median value of the respiratory muscle pressure within the set time period is used as the second target value. In some embodiments, the associated value refers to a maximum value, an average value or a median value of the respiratory muscle pressure itself. For example, a maximum value of the respiratory muscle pressure within a set time period is used as the second target value, or an average value of the respiratory muscle pressure within a set time period is used as the second target value. In other embodiments, the associated value refers to a value obtained by performing a certain calculation based on a maximum value, an average value or a median value of the respiratory muscle pressure. For example, after obtaining a maximum value of the respiratory muscle pressure, the maximum value is corrected by using a preset correction coefficient to obtain the second target value. For another example, after obtaining a maximum value of the respiratory muscle pressure, a preset value is added or subtracted to obtain the second target value.

In some embodiments, if the respiratory drive parameter is inspiratory work, the second target value is determined according to the inspiratory work which is obtained from an integral value over a set time period of the product of the respiratory muscle pressure and the airway flow rate. In other embodiments, N inspiratory work which corresponds to N respiratory cycles included in a set time period can also be obtained, wherein each respiratory cycle corresponds to one inspiratory work, and then an associated value of any one of a maximum value, an average value and a median value of the N inspiratory work is used as the second target value. In some embodiments, the associated value refers to a maximum value, an average value or a median value of the N inspiratory work itself. For example, a maximum value of the N inspiratory work is used as the second target value. In other embodiments, the associated value refers to a value obtained by performing a certain calculation based on a maximum value, an average value or a median value of N inspiratory work. For example, after obtaining an average value of N inspiratory work, the average value is corrected by using a preset correction coefficient to obtain the second target value. For another example, after obtaining an average value of N inspiratory work, a preset value is added or subtracted to obtain the first target value.

In some embodiments, if the respiratory drive parameter is a pressure area of respiratory muscle, the second target value is determined according to the pressure area of respiratory muscle which is obtained from an integral value over a set time period of the respiratory muscle pressure. In other embodiments, N pressure areas of respiratory muscle which correspond to N respiratory cycles included in a set time period can also be obtained, wherein each respiratory cycle corresponds to one pressure area of respiratory muscle. For example, in FIG. 5, N is 2, and there are two pressure areas of respiratory muscle. Then, an associated value of any one of a maximum value, an average value and a median value of the N pressure areas of respiratory muscle is used as the second target value. In some embodiments, the associated value refers to a maximum value, an average value or a median value of the N pressure areas of respiratory muscle itself. For example, a maximum value of the N pressure areas of respiratory muscle is used as the second target value. In other embodiments, the associated value refers to a value obtained by performing a certain calculation based on a maximum value, an average value or a median value of N pressure areas of respiratory muscle. For example, after obtaining an average value of N pressure areas of respiratory muscle, the average value is corrected by using a preset correction coefficient to obtain the second target value. For another example, after obtaining an average value of N pressure areas of respiratory muscle, a preset value is added or subtracted to obtain the second target value.

In some embodiments, if the respiratory drive parameter is a flow amount index, N flow amount indices which correspond to N respiratory cycles included in a set time period are calculated, and then an associated value of any one of a maximum value, an average value and a median value of the N flow amount indices is used as the second target value. In some embodiments, the associated value refers to a maximum value, an average value or a median value of the N flow amount indices itself. For example, a maximum value of the N flow amount indices is used as the second target value. In other embodiments, the associated value refers to a value obtained by performing a certain calculation based on a maximum value, an average value or a median value of N flow amount indices. For example, after obtaining an average value of N flow amount indices, the average value is corrected by using a preset correction coefficient to obtain the second target value. For another example, after obtaining an average value of N flow amount indices, a preset value is added or subtracted to obtain the second target value.

Step 4c: obtain, according to the second target value, a reference value which corresponds to a ventilation support parameter of the ventilation device; wherein a preset proportional relationship exists between the second target value and a parameter value which corresponds to the ventilation support parameter. It can be understood that proportional relationships between the parameter value of the ventilation support parameter in different types and the second target value can be the same or different. For example, when the ventilation support parameter is a support pressure, the proportional relationship can be x, and when the ventilation support parameter is a tidal volume, the proportional relationship can be y.

Step 5c: adjust and/or prompt to adjust, according to the reference value, a parameter value of the ventilation support parameter of the ventilation device in a target respiratory cycle. Wherein, the target respiratory cycle is after the set time period. That is to say, the ventilation regulation and control method shown in FIG. 9 determines a second target value through the parameter value(s) of the respiratory drive parameter in one or several respiratory cycles before the target respiratory cycle, and then proportionally adjusts the parameter value of the ventilation support parameter in the target respiratory cycle according to the second target value, so that the parameter value of the ventilation support parameter in the target respiratory cycle is in a preset proportion to the obtained second target value.

The currently implemented PAV (Proportional Assist Ventilation) mode is that: determining a ventilation pressure at a next moment according to a preset ratio based on an acquired real-time parameter value which represents a respiratory effort, and controlling the ventilation device according to the determined ventilation pressure at the next moment; specifically, within one respiratory cycle, the greater the parameter value of the respiratory effort at the i-th moment, the greater the support pressure given by the ventilator at the i+1 moment. Compared with the existing PAV mode, the embodiment described in FIG. 9 above, is as follows. On the one hand, the preset proportional relationship is a ratio between the second target value, which is obtained after a statistical analysis of the parameter value of the respiratory drive parameter, and the ventilation support parameter, while the PAV mode uses a ratio between the parameter value of the respiratory muscle pressure and the ventilation pressure, and the PAV mode has not undergone a statistical analysis. On the other hand, the PAV mode directly performs a proportional adjustment by acquiring a parameter value of the respiratory effort in real time, while this disclosure performs a statistical analysis based on the parameter value of the respiratory drive parameter, and only perform the steps of determining the parameter value corresponding to the ventilation support parameter after obtaining the second target value. Since the ventilation pressure adjusted by the real-time proportional adjustment method has a large fluctuation, it affects the stable regulation of the patient. This disclosure does not adopt a real-time regulation of the ventilation support parameter, which reduces a calculation complexity while achieving stable regulation of the patient.

In the above embodiments, the target value is determined by the parameter value of the respiratory drive parameter, and the parameter value of the ventilation support parameter is adjusted and/or prompted to be adjusted according to the target value, or the medication that affects the respiratory effort is adjusted and/or prompted to be adjusted according to the target value, thereby preventing diaphragm fatigue and diaphragm disuse atrophy.

Those skilled in the art will appreciate that all or part of functions of various methods in the above-mentioned implementations may be implemented by a hardware or by a computer program. When all or part of the functions in the above implementations are implemented by means of a computer program, the program can be stored in a computer-readable storage medium, and the storage medium may include: a read-only memory, a random access memory, a disk, a CD-ROM, a hard disk, etc., and the program is capable of being executed by a computer to implement all or part of above functions. For example, the program is stored in a memory of a device, and when the program in the memory is executed by a processor, all or part of the above functions can be realized. In addition, when all or part of the functions in the above-mentioned embodiments are implemented by means of a computer program, the program can also be stored in a storage medium, such as a server, another computer, a disk, an optical disk, a flash drive or a mobile hard disk, and can be saved to a memory of a local device by downloading or copying, or updating a version of a system of the local device. When the program in the memory is executed by the processor, all or part of the functions in the above-mentioned embodiments can be implemented.

The above specific examples are used to illustrate this disclosure, which are only used to help understand this disclosure and are not intended to limit this disclosure. For those skilled in the art, the above specific implementations may be modified according to the concept of this disclosure.

## Claims

1. A ventilation regulation and control method, **characterized in that**, comprising:

   obtaining a monitoring pressure and/or an airway flow rate of a ventilation device during mechanical ventilation for a patient;
   determining a parameter value of a respiratory drive parameter according to the monitoring pressure and/or the airway flow rate, wherein the respiratory drive parameter is for characterizing a respiratory effort of the patient, and comprises a respiratory muscle pressure, inspiratory work or a flow amount index; wherein the respiratory muscle pressure is calculated according to the monitoring pressure and/or the airway flow rate; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;
   performing a statistical analysis on the parameter value of the respiratory drive parameter, so as to determine a first target value; and
   comparing the first target value with a preset first range, and adjusting and/or prompting to adjust a parameter value of a ventilation support parameter according to a comparison result, wherein the ventilation support parameter is for reflecting a pressure or a flow amount condition which is set by the ventilation device.

2. The method according to claim 1, **characterized in that**:

if the respiratory drive parameter is the respiratory muscle pressure, performing a statistical analysis on the parameter value of the respiratory drive parameter, so as to determine a first target value, comprises:
obtaining the respiratory muscle pressure within a first set time period, and using as the first target value an associated value of any one of a maximum value, an average value and a median value of the respiratory muscle pressure within the first set time period.

3.  The method according to claim 2, **characterized in that**, the first set time period is a time period of at least one respiratory cycle.

4.  The method according to claim 1, **characterized in that**:
if the respiratory drive parameter is the inspiratory work, performing a statistical analysis on the parameter value of the respiratory drive parameter, so as to determine a first target value, comprises:
using as the first target value the inspiratory work which is obtained from an integral value over a second set time period of the product of the respiratory muscle pressure and the airway flow rate.

5.  The method according to claim 4, **characterized in that**, the second set time period is a time period of at least one respiratory cycle.

6.  The method according to claim 1, **characterized in that**:
if the respiratory drive parameter is the inspiratory work, performing a statistical analysis on the parameter value of the respiratory drive parameter, so as to determine a first target value, comprises:

    obtaining N inspiratory work which corresponds to N respiratory cycles, wherein each respiratory cycle corresponds to one inspiratory work; and using as the first target value an associated value of any one of a maximum value, an average value and a median value of the N inspiratory work;
    wherein N is an integer greater than or equal to 1.

7.  The method according to claim 1, **characterized in that**:
if the respiratory drive parameter is the flow amount index, performing a statistical analysis on the parameter value of the respiratory drive parameter, so as to determine a first target value, comprises:

    calculating N flow amount indexes which correspond to N respiratory cycles, wherein each respiratory cycle corresponds to one flow amount index; and using as the first target value an associated value of any one of a maximum value, an average value and a median value of the N flow amount indexes;
    wherein N is an integer greater than or equal to 1.

8.  The method according to any one of claims 1~7, **characterized in that**, adjusting and/or prompting to adjust a parameter value of a ventilation support parameter according to a comparison result, comprises:

    increasing or prompting to increase the parameter value of the ventilation support parameter, if the first target value is greater than an upper limit of the first range; and/or
    decreasing or prompting to decrease the parameter value of the ventilation support parameter, if the first target value is less than a lower limit of the first range.

9.  The method according to claim 8, **characterized in that**:
increasing or prompting to increase the parameter value of the ventilation support parameter, comprises:

    increasing the parameter value of the ventilation support parameter according to a first adjustment step which corresponds to the ventilation support parameter; and/or prompting to increase the parameter value of the ventilation support parameter according to a first adjustment step which corresponds to the ventilation support parameter;
    decreasing or prompting to decrease the parameter value of the ventilation support parameter, comprises:
    decreasing the parameter value of the ventilation support parameter according to a second adjustment step which corresponds to the ventilation support parameter; and/or prompting to decrease the parameter value of the ventilation support parameter according to a second adjustment step which corresponds to the ventilation support parameter.

10. The method according to any one of claims 1~9, **characterized in that**, determining a parameter value of a respiratory drive parameter according to the monitoring pressure and/or the airway flow rate, comprises:

when the respiratory drive parameter is the respiratory muscle pressure and the monitoring pressure is an airway pressure of the patient, calculating the respiratory muscle pressure according to a preset respiratory mechanics equation, the airway pressure and the airway flow rate; or

when the respiratory drive parameter is the inspiratory work and the monitoring pressure is an airway pressure of the patient, calculating the respiratory muscle pressure according to a preset respiratory mechanics equation, the airway pressure and the airway flow rate, and using as the inspiratory work the integral value over time of the product of the respiratory muscle pressure and the airway flow rate.

11. The method according to claim 10, **characterized in that**, calculating the respiratory muscle pressure according to a preset respiratory mechanics equation, the airway pressure and the airway flow rate, comprises:

respectively substituting multiple groups of the airway pressure, the airway flow rate and a ventilation volume, which groups are at different time points, into the preset respiratory mechanics equation, so as to obtain an equation group of the respiratory muscle pressure and a total positive end-expiratory pressure;

solving the equation group to calculate the respiratory muscle pressure;

wherein, the preset respiratory mechanics equation is:

$$\text{Paw-Pmus=Flow} * \text{R+Volume} * \text{E+PEEPtot} ;$$

wherein **Paw** represents the airway pressure, **Pmus** represents the respiratory muscle pressure, **Flow** represents the airway flow rate, **Volume** represents the ventilation volume which is obtained according to an integral value over time of the airway flow rate; **R** represents a viscous resistance of a respiratory system of the patient, **E** represents an elastic resistance of the respiratory system of the patient, **PEEPtot** represents the total positive end-expiratory pressure.

12. The method according to any one of claims 1~9, **characterized in that**, determining a parameter value of a respiratory drive parameter according to the monitoring pressure and/or the airway flow rate, comprises:

when the respiratory drive parameter is the respiratory muscle pressure and the monitoring pressure is an esophageal pressure of the patient, calculating chest wall compliance of the patient according to the esophageal pressure and the airway flow rate; and calculating the respiratory muscle pressure according to a human respiratory system model through the airway flow rate, the esophageal pressure and the chest wall compliance; or

when the respiratory drive parameter is the inspiratory work and the monitoring pressure is an esophageal pressure of the patient, calculating chest wall compliance of the patient according to the esophageal pressure and the airway flow rate; calculating the respiratory muscle pressure according to a human respiratory system model through the airway flow rate, the esophageal pressure and the chest wall compliance; and using as the inspiratory work the integral value over time of the product of the respiratory muscle pressure and the airway flow rate.

13. The method according to any one of claims 1~9, **characterized in that**, determining a parameter value of a respiratory drive parameter according to the monitoring pressure and/or the airway flow rate, comprises:

when the respiratory drive parameter is the respiratory muscle pressure and the monitoring pressure is an esophageal pressure and an intragastric pressure of the patient, calculating a transdiaphragmatic pressure of the patient according to the esophageal pressure and the intragastric pressure, and using the transdiaphragmatic pressure as the respiratory muscle pressure; or

when the respiratory drive parameter is the inspiratory work and the monitoring pressure is an esophageal pressure and an intragastric pressure of the patient, calculating a transdiaphragmatic pressure of the patient according to the esophageal pressure and the intragastric pressure, using the transdiaphragmatic pressure as the respiratory muscle pressure, and using as the inspiratory work the integral value over time of the product of the respiratory muscle pressure and the airway flow rate.

14. The method according to any one of claims 1~9, **characterized in that**, determining a parameter value of a respiratory drive parameter according to the monitoring pressure and/or the airway flow rate, comprises:

when the respiratory drive parameter is the flow amount index, calculating the flow amount index which corresponds to one respiratory cycle according to following formula:

$$F = a + b\Delta t^{c};$$

wherein, **F** represents the airway flow rate in an inspiratory phase of the respiratory cycle, **a** represents a peak value of the airway flow rate in the inspiratory phase of the respiratory cycle, **b** represents a reduction rate of flow amount which rate corresponds to a selected time period $\Delta t$ in the inspiratory phase of the respiratory cycle, and c represents the flow amount index.

15. The method according to any one of claims 1~14, **characterized in that**, the ventilation support parameter at least comprises one of: a support pressure, an inspiratory pressure, a tidal volume, and a ventilation mount per minute.

16. A ventilation regulation and control method, **characterized in that**, comprising:

obtaining a monitoring pressure and/or an airway flow rate of a ventilation device during mechanical ventilation for a patient;
determining a parameter value of a respiratory drive parameter according to the monitoring pressure and/or the airway flow rate, wherein the respiratory drive parameter is for characterizing a respiratory effort of the patient, and comprises a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;
performing a statistical analysis on the parameter value of the respiratory drive parameter, so as to determine a first target value;
comparing the first target value with a preset first range, and adjusting and/or prompting to adjust a medication which affects the respiratory effort according to a comparison result.

17. The method according to claim 16, **characterized in that**:
if the respiratory drive parameter is the respiratory muscle pressure, performing a statistical analysis on the parameter value of the respiratory drive parameter, so as to determine a first target value, comprises:
obtaining the respiratory muscle pressure within a first set time period, and using as the first target value an associated value of any one of a maximum value, an average value and a median value of the respiratory muscle pressure within the first set time period.

18. The method according to claim 17, **characterized in that**, the first set time period is a time period of at least one respiratory cycle.

19. The method according to claim 16, **characterized in that**:
if the respiratory drive parameter is the pressure area of respiratory muscle, performing a statistical analysis on the parameter value of the respiratory drive parameter, so as to determine a first target value, comprises:
using as the first target value the pressure area of respiratory muscle which is obtained from an integral value over a third set time period of the respiratory muscle pressure.

20. The method according to claim 19, **characterized in that**, the third set time period is a time period of at least one respiratory cycle.

21. The method according to claim 16, **characterized in that**:
if the respiratory drive parameter is the pressure area of respiratory muscle, performing a statistical analysis on the parameter value of the respiratory drive parameter, so as to determine a first target value, comprises:

obtaining N pressure areas of respiratory muscle which correspond to N respiratory cycles, wherein each respiratory cycle corresponds to one pressure area of respiratory muscle; using as the first target value an associated value of any one of a maximum value, an average value and a median value of the N pressure areas of respiratory muscle;

wherein N is an integer greater than or equal to 1.

22. The method according to claim 16, **characterized in that**:
if the respiratory drive parameter is the inspiratory work, performing a statistical analysis on the parameter value of the respiratory drive parameter, so as to determine a first target value, comprises:
using as the first target value the inspiratory work which is obtained from an integral value over a second set time period of the product of the respiratory muscle pressure and the airway flow rate.

23. The method according to claim 22, **characterized in that**, the second set time period is a time period of at least one respiratory cycle.

24. The method according to claim 16, **characterized in that**:
if the respiratory drive parameter is the inspiratory work, performing a statistical analysis on the parameter value of the respiratory drive parameter, so as to determine a first target value, comprises:

obtaining N inspiratory work which corresponds to N respiratory cycles, wherein each respiratory cycle corresponds to one inspiratory work; and using as the first target value an associated value of any one of a maximum value, an average value and a median value of the N inspiratory work;
wherein N is an integer greater than or equal to 1.

25. The method according to claim 16, **characterized in that**:
if the respiratory drive parameter is the flow amount index, performing a statistical analysis on the parameter value of the respiratory drive parameter, so as to determine a first target value, comprises:

calculating N flow amount indexes which correspond to N respiratory cycles, wherein each respiratory cycle corresponds to one flow amount index; and using as the first target value an associated value of any one of a maximum value, an average value and a median value of the N flow amount indexes;
wherein N is an integer greater than or equal to 1.

26. The method according to claim 16, **characterized in that**, adjusting and/or prompting to adjust a medication which affects the respiratory effort according to a comparison result, comprises:

increasing or prompting to increase the medication which affects the respiratory effort, if the first target value is greater than an upper limit of the first range; and/or
decreasing or prompting to decrease the medication which affects the respiratory effort, if the first target value is less than a lower limit of the first range.

27. The method according to claim 26, **characterized in that**, increasing or prompting to increase the medication which affects the respiratory effort, comprises:

obtaining a first difference between the first target value and the upper limit of the first range, determining first medication administration data according to the first difference, and increasing and/or prompting to increase the medication which affects the respiratory effort according to the first medication administration data;
wherein, the first medication administration data comprise a first medication administration dosage for the medication which affects the respiratory effort, and/or a first medication administration frequency of a preset dosage for the medication which affects the respiratory effort;
wherein the first medication administration dosage is greater than a medication administration dosage before adjusting, the first medication administration frequency is higher than a medication administration frequency of the preset dosage before adjusting.

28. The method according to claim 26, **characterized in that**, decreasing or prompting to decrease the medication which affects the respiratory effort, comprises:

obtaining a second difference between the first target value and the lower limit of the first range, determining second medication administration data according to the second difference, and decreasing and/or prompting to decrease the medication which affects the respiratory effort according to the second medication administration data;
wherein, the second medication administration data comprise a second medication administration dosage for the medication which affects the respiratory effort, and/or a second medication administration frequency of a preset dosage for the medication which affects the respiratory effort;

wherein the second medication administration dosage is less than a medication administration dosage before adjusting, the second medication administration frequency is lower than a medication administration frequency of the preset dosage before adjusting.

29. The method according to any one of claims 16-24, or 26~28, **characterized in that**, determining a parameter value of a respiratory drive parameter according to the monitoring pressure and/or the airway flow rate, comprises:

when the respiratory drive parameter is the respiratory muscle pressure and the monitoring pressure is an airway pressure of the patient, calculating the respiratory muscle pressure according to a preset respiratory mechanics equation, the airway pressure and the airway flow rate; or

when the respiratory drive parameter is the pressure area of respiratory muscle and the monitoring pressure is an airway pressure of the patient, calculating the respiratory muscle pressure according to a preset respiratory mechanics equation, the airway pressure and the airway flow rate, and using as the pressure area of respiratory muscle the integral value over time of the respiratory muscle pressure; or

when the respiratory drive parameter is the inspiratory work and the monitoring pressure is an airway pressure of the patient, calculating the respiratory muscle pressure according to a preset respiratory mechanics equation, the airway pressure and the airway flow rate, and using as the inspiratory work the integral value over time of the product of the respiratory muscle pressure and the airway flow rate.

30. The method according to claim 29, **characterized in that**, calculating the respiratory muscle pressure according to a preset respiratory mechanics equation, the airway pressure and the airway flow rate, comprises:

respectively substituting multiple groups of the airway pressure, the airway flow rate and a ventilation volume, which groups are at different time points, into the preset respiratory mechanics equation, so as to obtain an equation group of the respiratory muscle pressure and a total positive end-expiratory pressure;

solving the equation group to calculate the respiratory muscle pressure;

wherein, the preset respiratory mechanics equation is:

$$\text{Paw-Pmus=Flow} * \text{R+Volume} * \text{E+PEEPtot} ;$$

wherein **Paw** represents the airway pressure, **Pmus** represents the respiratory muscle pressure, **Flow** represents the airway flow rate, **Volume** represents the ventilation volume which is obtained according to an integral value over time of the airway flow rate; **R** represents a viscous resistance of a respiratory system of the patient, **E** represents an elastic resistance of the respiratory system of the patient, **PEEPtot** represents the total positive end-expiratory pressure.

31. The method according to any one of claims 16-24, or 26~28, **characterized in that**, determining a parameter value of a respiratory drive parameter according to the monitoring pressure and/or the airway flow rate, comprises:

when the respiratory drive parameter is the respiratory muscle pressure and the monitoring pressure is an esophageal pressure of the patient, calculating chest wall compliance of the patient according to the esophageal pressure and the airway flow rate; and calculating the respiratory muscle pressure according to a human respiratory system model through the airway flow rate, the esophageal pressure and the chest wall compliance; or

when the respiratory drive parameter is the pressure area of respiratory muscle and the monitoring pressure is an esophageal pressure of the patient, calculating chest wall compliance of the patient according to the esophageal pressure and the airway flow rate; calculating the respiratory muscle pressure according to a human respiratory system model through the airway flow rate, the esophageal pressure and the chest wall compliance; and using as the pressure area of respiratory muscle the integral value over time of the respiratory muscle pressure;

when the respiratory drive parameter is the inspiratory work and the monitoring pressure is an esophageal pressure of the patient, calculating chest wall compliance of the patient according to the esophageal pressure and the airway flow rate; calculating the respiratory muscle pressure according to a human respiratory system model through the airway flow rate, the esophageal pressure and the chest wall compliance; and using as the inspiratory work the integral value over time of the product of the respiratory muscle pressure and the airway flow rate.

32. The method according to any one of claims 16-24, or 26~28, **characterized in that**, determining a parameter value of a respiratory drive parameter according to the monitoring pressure and/or the airway flow rate, comprises:

when the respiratory drive parameter is the respiratory muscle pressure and the monitoring pressure is an esophageal pressure and an intragastric pressure of the patient, calculating a transdiaphragmatic pressure of the patient according to the esophageal pressure and the intragastric pressure, and using the transdiaphragmatic pressure as the respiratory muscle pressure; or
when the respiratory drive parameter is the pressure area of respiratory muscle and the monitoring pressure is an esophageal pressure and an intragastric pressure of the patient, calculating a transdiaphragmatic pressure of the patient according to the esophageal pressure and the intragastric pressure, using the transdiaphragmatic pressure as the respiratory muscle pressure, and further using as the pressure area of respiratory muscle the integral value over time of the respiratory muscle pressure;
when the respiratory drive parameter is the inspiratory work and the monitoring pressure is an esophageal pressure and an intragastric pressure of the patient, calculating a transdiaphragmatic pressure of the patient according to the esophageal pressure and the intragastric pressure, using the transdiaphragmatic pressure as the respiratory muscle pressure, and using as the inspiratory work the integral value over time of the product of the respiratory muscle pressure and the airway flow rate.

33. The method according to any one of claims 16 or 25~28, **characterized in that**, determining a parameter value of a respiratory drive parameter according to the monitoring pressure and/or the airway flow rate, comprises:

when the respiratory drive parameter is the flow amount index, calculating the flow amount index which corresponds to one respiratory cycle according to following formula:

$$F = a + b\Delta t^{c};$$

wherein, **F** represents the airway flow rate in an inspiratory phase of the respiratory cycle, **a** represents a peak value of the airway flow rate in the inspiratory phase of the respiratory cycle, **b** represents a reduction rate of flow amount which rate corresponds to a selected time period $\Delta$**t** in the inspiratory phase of the respiratory cycle, and c represents the flow amount index.

34. A ventilation regulation and control method, **characterized in that**, comprising:

obtaining a monitoring pressure and/or an airway flow rate of a ventilation device during mechanical ventilation for a patient;
determining a parameter value of a respiratory drive parameter according to the monitoring pressure and/or the airway flow rate, wherein the respiratory drive parameter is for characterizing a respiratory effort of the patient, and comprises a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;
performing a statistical analysis on the parameter value of the respiratory drive parameter within a set time period, so as to determine a second target value, wherein the set time period comprises N respiratory cycles, wherein N is an integer greater than or equal to 1;
according to a preset proportional relationship between a target value which corresponds to the respiratory drive parameter and a parameter value which corresponds to a ventilation support parameter, obtaining, according to the second target value, a reference value which corresponds to a ventilation support parameter of the ventilation device; and
adjusting and/or prompting to adjust, according to the reference value, a parameter value of the ventilation support parameter of the ventilation device in a target respiratory cycle; wherein the target respiratory cycle is after the set time period.

35. The method according to claim 34, **characterized in that**:
if the respiratory drive parameter is the respiratory muscle pressure, performing a statistical analysis on the parameter value of the respiratory drive parameter within a set time period, so as to determine a second target value, comprises:

obtaining the respiratory muscle pressure within the set time period, and using as the second target value an associated value of any one of a maximum value, an average value and a median value of the respiratory muscle pressure within the set time period.

36. The method according to claim 34, **characterized in that**:
if the respiratory drive parameter is the pressure area of respiratory muscle, performing a statistical analysis on the parameter value of the respiratory drive parameter within a set time period, so as to determine a second target value, comprises:

using as the second target value the pressure area of respiratory muscle which is obtained from the integral value over the set time period of the respiratory muscle pressure; or
obtaining N pressure areas of respiratory muscle which correspond to N respiratory cycles which respiratory cycles are included in the set time period, wherein each respiratory cycle corresponds to one pressure area of respiratory muscle; using as the second target value an associated value of any one of a maximum value, an average value and a median value of the N pressure areas of respiratory muscle.

37. The method according to claim 34, **characterized in that**:
if the respiratory drive parameter is the inspiratory work, performing a statistical analysis on the parameter value of the respiratory drive parameter within a set time period, so as to determine a second target value, comprises:

using as the second target value the inspiratory work which is obtained from the integral value over the set time period of the product of the respiratory muscle pressure and the airway flow rate; or
obtaining N inspiratory work which corresponds to N respiratory cycles which respiratory cycles are included in the set time period, wherein each respiratory cycle corresponds to one inspiratory work; using as the second target value an associated value of any one of a maximum value, an average value and a median value of the N inspiratory work.

38. The method according to claim 34, **characterized in that**:
if the respiratory drive parameter is the flow amount index, performing a statistical analysis on the parameter value of the respiratory drive parameter within a set time period, so as to determine a second target value, comprises:
calculating N flow amount indexes which correspond to N respiratory cycles which respiratory cycles are included in the set time period, wherein each respiratory cycle corresponds to one flow amount index; using as the second target value an associated value of any one of a maximum value, an average value and a median value of the N flow amount indexes.

39. The method according to any one of claims 34~37, **characterized in that**, determining a parameter value of a respiratory drive parameter according to the monitoring pressure and/or the airway flow rate, comprises:

when the respiratory drive parameter is the respiratory muscle pressure and the monitoring pressure is an airway pressure of the patient, calculating the respiratory muscle pressure according to a preset respiratory mechanics equation, the airway pressure and the airway flow rate; or
when the respiratory drive parameter is the pressure area of respiratory muscle and the monitoring pressure is an airway pressure of the patient, calculating the respiratory muscle pressure according to a preset respiratory mechanics equation, the airway pressure and the airway flow rate, and using as the pressure area of respiratory muscle the integral value over time of the respiratory muscle pressure; or
when the respiratory drive parameter is the inspiratory work and the monitoring pressure is an airway pressure of the patient, calculating the respiratory muscle pressure according to a preset respiratory mechanics equation, the airway pressure and the airway flow rate, and using as the inspiratory work the integral value over time of the product of the respiratory muscle pressure and the airway flow rate.

40. The method according to claim 39, **characterized in that**, calculating the respiratory muscle pressure according to a preset respiratory mechanics equation, the airway pressure and the airway flow rate, comprises:

respectively substituting multiple groups of the airway pressure, the airway flow rate and a ventilation volume, which groups are at different time points, into the preset respiratory mechanics equation, so as to obtain an equation group of the respiratory muscle pressure and a total positive end-expiratory pressure;
solving the equation group to calculate the respiratory muscle pressure;
wherein, the preset respiratory mechanics equation is:

$$Paw-Pmus=Flow*R+Volume*E+PEEPtot\ ;$$

wherein **Paw** represents the airway pressure, **Pmus** represents the respiratory muscle pressure, **Flow** represents the airway flow rate, **Volume** represents the ventilation volume which is obtained according to an integral value over time of the airway flow rate; **R** represents a viscous resistance of a respiratory system of the patient, **E** represents an elastic resistance of the respiratory system of the patient, **PEEPtot** represents the total positive end-expiratory pressure.

41. The method according to any one of claims 34~37, **characterized in that**, determining a parameter value of a respiratory drive parameter according to the monitoring pressure and/or the airway flow rate, comprises:

when the respiratory drive parameter is the respiratory muscle pressure and the monitoring pressure is an esophageal pressure of the patient, calculating chest wall compliance of the patient according to the esophageal pressure and the airway flow rate; and calculating the respiratory muscle pressure according to a human respiratory system model through the airway flow rate, the esophageal pressure and the chest wall compliance; or
when the respiratory drive parameter is the pressure area of respiratory muscle and the monitoring pressure is an esophageal pressure of the patient, calculating chest wall compliance of the patient according to the esophageal pressure and the airway flow rate; calculating the respiratory muscle pressure according to a human respiratory system model through the airway flow rate, the esophageal pressure and the chest wall compliance; and using as the pressure area of respiratory muscle the integral value over time of the respiratory muscle pressure;
when the respiratory drive parameter is the inspiratory work and the monitoring pressure is an esophageal pressure of the patient, calculating chest wall compliance of the patient according to the esophageal pressure and the airway flow rate; calculating the respiratory muscle pressure according to a human respiratory system model through the airway flow rate, the esophageal pressure and the chest wall compliance; and using as the inspiratory work the integral value over time of the product of the respiratory muscle pressure and the airway flow rate.

42. The method according to any one of claims 34~37, **characterized in that**, determining a parameter value of a respiratory drive parameter according to the monitoring pressure and/or the airway flow rate, comprises:

when the respiratory drive parameter is the respiratory muscle pressure and the monitoring pressure is an esophageal pressure and an intragastric pressure of the patient, calculating a transdiaphragmatic pressure of the patient according to the esophageal pressure and the intragastric pressure, and using the transdiaphragmatic pressure as the respiratory muscle pressure; or
when the respiratory drive parameter is the pressure area of respiratory muscle and the monitoring pressure is an esophageal pressure and an intragastric pressure of the patient, calculating a transdiaphragmatic pressure of the patient according to the esophageal pressure and the intragastric pressure, using the transdiaphragmatic pressure as the respiratory muscle pressure, and further using as the pressure area of respiratory muscle the integral value over time of the respiratory muscle pressure;
when the respiratory drive parameter is the inspiratory work and the monitoring pressure is an esophageal pressure and an intragastric pressure of the patient, calculating a transdiaphragmatic pressure of the patient according to the esophageal pressure and the intragastric pressure, using the transdiaphragmatic pressure as the respiratory muscle pressure, and using as the inspiratory work the integral value over time of the product of the respiratory muscle pressure and the airway flow rate.

43. The method according to any one of claims 34 or 38, **characterized in that**, determining a parameter value of a respiratory drive parameter according to the monitoring pressure and/or the airway flow rate, comprises:

when the respiratory drive parameter is the flow amount index, calculating the flow amount index which corresponds to one respiratory cycle according to following formula:

$$F=a+b\Delta t^{c}\ ;$$

wherein, **F** represents the airway flow rate in an inspiratory phase of the respiratory cycle, **a** represents a peak value of the airway flow rate in the inspiratory phase of the respiratory cycle, **b** represents a reduction rate of flow amount which rate corresponds to a selected time period $\Delta$**t** in the inspiratory phase of the respiratory cycle, and c represents the flow amount index.

44. The method according to any one of claims 34~43, **characterized in that**, the ventilation support parameter at least comprises one of: a support pressure, an inspiratory pressure, a tidal volume, and a ventilation mount per minute.

45. A ventilation device, **characterized in that**, comprising:

a ventilation apparatus, which is configured to generate a gas according to a preset setting which at least comprises a ventilation support parameter;

a ventilation pipeline, which is connected with a respiratory system of a patient, so as to deliver the gas to the patient;

a sensor assembly, which at least comprises a pressure sensor and a flow amount sensor, wherein the pressure sensor is configured to acquire a monitoring pressure of the ventilation device during mechanical ventilation of the patient, the flow amount sensor is configured to acquire an airway flow rate of the ventilation device during the mechanical ventilation of the patient;

a processor, which is in respective signal connection with the ventilation apparatus and the sensor assembly;

the processor is configured to obtain the monitoring pressure acquired by the pressure sensor and/or the airway flow rate acquired by the flow amount sensor during the mechanical ventilation of the patient; and determine a parameter value of a respiratory drive parameter according to the monitoring pressure and/or the airway flow rate, wherein the respiratory drive parameter is for characterizing a respiratory effort of the patient, and comprises a respiratory muscle pressure, inspiratory work or a flow amount index; wherein the respiratory muscle pressure is calculated according to the monitoring pressure and/or the airway flow rate; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

the processor is further configured to perform a statistical analysis on the parameter value of the respiratory drive parameter, so as to determine a first target value, compare the first target value with a preset first range, and adjust and/or prompt to adjust a parameter value of a ventilation support parameter according to a comparison result, wherein the ventilation support parameter is for reflecting a pressure or a flow amount condition which is set by the ventilation device.

46. A ventilation device, **characterized in that**, comprising:

a ventilation apparatus, which is configured to generate a gas according to a set pressure or a set flow amount;

a ventilation pipeline, which is connected with a respiratory system of a patient, so as to deliver the gas to the patient;

a sensor assembly, which at least comprises a pressure sensor and a flow amount sensor, wherein the pressure sensor is configured to acquire a monitoring pressure of the ventilation device during mechanical ventilation of the patient, the flow amount sensor is configured to acquire an airway flow rate of the ventilation device during the mechanical ventilation of the patient;

a processor, which is in respective signal connection with the ventilation apparatus and the sensor assembly, and further in communicative connection with an infusion pump;

the processor is further configured to obtain the monitoring pressure and/or the airway flow rate of the ventilation device during the mechanical ventilation of the patient; determine a parameter value of a respiratory drive parameter according to the monitoring pressure and/or the airway flow rate, wherein the respiratory drive parameter is for characterizing a respiratory effort of the patient, and comprises a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

the processor is further configured to perform a statistical analysis on the parameter value of the respiratory drive parameter, so as to determine a first target value, compare the first target value with a preset first range, and control the infusion pump to adjust and/or prompt to adjust a medication which affects the respiratory effort according to a comparison result.

47. A ventilation device, **characterized in that**, comprising:

a ventilation apparatus, which is configured to generate a gas according to a preset setting which at least comprises ventilation support parameter;

a ventilation pipeline, which is connected with a respiratory system of a patient, so as to deliver the gas to the patient;

a sensor assembly, which at least comprises a pressure sensor and a flow amount sensor, wherein the pressure sensor is configured to acquire a monitoring pressure of the ventilation device during mechanical ventilation of the patient, the flow amount sensor is configured to acquire an airway flow rate of the ventilation device during the mechanical ventilation of the patient;

a processor, which is in respective signal connection with the ventilation apparatus and the sensor assembly; the processor is configured to obtain the monitoring pressure acquired by the pressure sensor and/or the airway flow rate acquired by the flow amount sensor during the mechanical ventilation of the patient; and determine a parameter value of a respiratory drive parameter according to the monitoring pressure and/or the airway flow rate, wherein the respiratory drive parameter is for characterizing a respiratory effort of the patient, and comprises a respiratory muscle pressure, a pressure area of respiratory muscle, inspiratory work or a flow amount index; wherein the respiratory muscle pressure is calculated according to the monitoring pressure and/or the airway flow rate; the pressure area of respiratory muscle is obtained according to an integral value over time of the respiratory muscle pressure; the inspiratory work is obtained according to an integral value over time of a product of the respiratory muscle pressure and the airway flow rate; the flow amount index is for characterizing a rate for a change within a descending process of an inspiratory flow amount curve, which curve is obtained from the airway flow rate;

the processor is further configured to perform a statistical analysis on the parameter value of the respiratory drive parameter within a set time period, so as to determine a second target value, wherein the set time period comprises N respiratory cycles, wherein N is an integer greater than or equal to 1;

the processor is further configured to: according to a preset proportional relationship between a target value which corresponds to the respiratory drive parameter and a parameter value which corresponds to a ventilation support parameter, obtain, according to the second target value, a reference value which corresponds to a ventilation support parameter of the ventilation device; adjust and/or prompt to adjust, according to the reference value, a parameter value of the ventilation support parameter of the ventilation device in a target respiratory cycle; wherein the target respiratory cycle is after the set time period.

**48.** A medical device, **characterized in that**, comprising:

a memory, which is configured to store a program;

a processor, which is configured to implement the method according to any one of claims 1~44 by executing the program stored in the memory.

**49.** A computer-readable storage medium, comprising a program, **characterized in that**, the program is capable of being executed by a processor, so as to implement the method according to any one of claims 1~44.

FIG. 1

Expiratory
branch 213a

213c

Patient

211

214a

Inspiratory
branch 213b

214b

212

G
a
s

| Memory 20 | Processor 10 | Display 30 |

| Pressure sensor 40 | | Flow amount sensor 50 |

FIG. 2

Expiratory
branch 340a

Patient

311

340c

Inspiratory
branch 340b

Anaesthetic output apparatus

330

Respiration assistance apparatus

320

312

G
a
s

| Memory 20 | Processor 10 | Display 30 |

| Pressure sensor 40 | | Flow amount sensor 50 |

FIG. 3

Obtain a monitoring pressure and/or an airway flow rate during mechanical ventilation for a patient

1a

Determine a parameter value of a respiratory drive parameter according to the monitoring pressure and/or the airway flow rate

2a

Perform a statistical analysis on the parameter value of the respiratory drive parameter, so as to determine a first target value

3a

Compare the first target value with a preset first range

4a

Adjust and/or prompt to adjust a parameter value of a ventilation support parameter according to a comparison result

5a

FIG. 4

Airway pressure

Paw

Pmus

Time

Airway flow rate

Flow

Time

Ventilation volume

Volume

Time

FIG. 5

FIG. 6

FIG. 7

Obtain a monitoring pressure and/or an airway flow rate during mechanical ventilation for a patient

1a

Determine a parameter value of a respiratory drive parameter according to the monitoring pressure and/or the airway flow rate

2a

Perform a statistical analysis on the parameter value of the respiratory drive parameter, so as to determine a first target value

3a

Compare the first target value with a preset first range

4a

Control an infusion pump to adjust and/or prompt to adjust a medication that affects the respiratory effort, according to a comparison result

5b

FIG. 8

Obtain a monitoring pressure and/or an airway flow rate during mechanical ventilation for a patient

1a

Determine a parameter value of a respiratory drive parameter according to the monitoring pressure and/or the airway flow rate

2a

Perform a statistical analysis on the parameter value of the respiratory drive parameter within a set time period, so as to determine a second target value

3c

Obtain, according to the second target value, a reference value which corresponds to a ventilation support parameter of the ventilation device

4c

Adjust and/or prompt to adjust, according to the reference value, a parameter value of the ventilation support parameter of the ventilation device in a target respiratory cycle

5c

FIG. 9

## INTERNATIONAL SEARCH REPORT

| | |
|---|---|
| International application No. | |
| | **PCT/CN2023/072158** |

**A.    CLASSIFICATION OF SUBJECT MATTER**

A61M 16/00(2006.01)i;  A61B 5/087(2006.01)i

According to International Patent Classification (IPC) or to both national classification and IPC

**B.    FIELDS SEARCHED**

Minimum documentation searched (classification system followed by classification symbols)

IPC: A61M 16, A61B 5

Documentation searched other than minimum documentation to the extent that such documents are included in the fields searched

Electronic data base consulted during the international search (name of data base and, where practicable, search terms used)

CNTXT, ENTXT, ENTXTC, VEN: 波形, 调节, 反馈, 峰值, 呼吸, 呼吸道压力, 呼吸肌压力, 肌肉压力, 气道压力, 食道压力, 积分, 流量, 流量指数, 努力, 平均, 通气, 统计, 吸气功, 呼吸功, Pmus, POB, WOB

**C.    DOCUMENTS CONSIDERED TO BE RELEVANT**

| Category* | Citation of document, with indication, where appropriate, of the relevant passages | Relevant to claim No. |
|---|---|---|
| X | US 6390091 B1 (UNIVERSITY OF FLORIDA) 21 May 2002 (2002-05-21) description, column 7, line 15 to column 26, line 24, and figures 1-12 | 1-3, 8-10, 12, 13, 15-21, 26-29, 31, 32, 34-36, 39, 41, 42, 44-49 |
| Y | US 6390091 B1 (UNIVERSITY OF FLORIDA) 21 May 2002 (2002-05-21) description, column 7, line 15 to column 26, line 24, and figures 1-12 | 4-7, 11, 14, 22-25, 30, 33, 37, 38, 40, 43 |
| Y | CN 109906054 A (KONINKL PHILIPS N.V.) 18 June 2019 (2019-06-18) description, paragraphs 3-68, and figures 1-5 | 4-6, 11, 22-24, 30, 37, 40 |
| Y | US 5803066 A (NEW YORK UNIVERSITY) 08 September 1998 (1998-09-08) description, column 9, lines 36-46 | 7, 14, 25, 33, 38, 43 |
| A | CN 108135536 A (KONINKL PHILIPS N.V.) 08 June 2018 (2018-06-08) entire document | 1-49 |
| A | CN 115486832 A (DRAEGERWERK AG AND CO. KGAA) 20 December 2022 (2022-12-20) entire document | 1-49 |
| A | US 2021016035 A1 (CONVERGENT ENGINEERING, INC.) 21 January 2021 (2021-01-21) entire document | 1-49 |

☑ Further documents are listed in the continuation of Box C.     ☑ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **26 September 2023** | **08 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/072158** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 108348718 A (KONINKL PHILIPS N.V.) 31 July 2018 (2018-07-31)<br>entire document | 1-49 |
| A | US 2009159082 A1 (DRAGER MEDICAL AG & CO. KG) 25 June 2009 (2009-06-25)<br>entire document | 1-49 |

Form PCT/ISA/210 (second sheet) (July 2022)

## INTERNATIONAL SEARCH REPORT
### Information on patent family members

| International application No. |
|---|
| **PCT/CN2023/072158** |

| Patent document cited in search report | | | Publication date (day/month/year) | Patent family member(s) | | | Publication date (day/month/year) |
|---|---|---|---|---|---|---|---|
| US | 6390091 | B1 | 21 May 2002 | AU | 2866500 | A | 25 August 2000 |
| | | | | CA | 2362160 | A1 | 10 August 2000 |
| | | | | GB | 0116912 | D0 | 05 September 2001 |
| | | | | GB | 2362109 | A | 14 November 2001 |
| | | | | NZ | 513864 | A | 28 September 2001 |
| | | | | WO | 0045880 | A1 | 10 August 2000 |
| | | | | WO | 0045880 | A9 | 02 May 2002 |
| | | | | EP | 1173246 | A1 | 23 January 2002 |
| CN | 109906054 | A | 18 June 2019 | WO | 2018078505 | A1 | 03 May 2018 |
| | | | | JP | 2020503079 | A | 30 January 2020 |
| | | | | JP | 7168560 | B2 | 09 November 2022 |
| | | | | US | 2019254566 | A1 | 22 August 2019 |
| | | | | EP | 3544502 | A1 | 02 October 2019 |
| US | 5803066 | A | 08 September 1998 | WO | 9728838 | A1 | 14 August 1997 |
| | | | | JP | 2007301372 | A | 22 November 2007 |
| | | | | JP | 4464987 | B2 | 19 May 2010 |
| | | | | JP | 2000504602 | A | 18 April 2000 |
| | | | | JP | 4023826 | B2 | 19 December 2007 |
| | | | | DE | 69731899 | D1 | 13 January 2005 |
| | | | | DE | 69731899 | T2 | 24 November 2005 |
| | | | | ES | 2234001 | T3 | 16 June 2005 |
| | | | | EP | 0956066 | A1 | 17 November 1999 |
| | | | | EP | 0956066 | A4 | 31 January 2001 |
| | | | | EP | 0956066 | B1 | 08 December 2004 |
| | | | | AU | 1953597 | A | 28 August 1997 |
| | | | | AU | 722608 | B2 | 10 August 2000 |
| | | | | AU | 722608 | C | 21 September 2006 |
| | | | | AT | 284233 | T | 15 December 2004 |
| CN | 108135536 | A | 08 June 2018 | EP | 3356948 | A1 | 08 August 2018 |
| | | | | EP | 3356948 | B1 | 16 February 2022 |
| | | | | WO | 2017055959 | A1 | 06 April 2017 |
| | | | | US | 2018279963 | A1 | 04 October 2018 |
| | | | | US | 11191441 | B2 | 07 December 2021 |
| | | | | JP | 2018536510 | A | 13 December 2018 |
| | | | | JP | 6876057 | B2 | 26 May 2021 |
| CN | 115486832 | A | 20 December 2022 | US | 2022401674 | A1 | 22 December 2022 |
| US | 2021016035 | A1 | 21 January 2021 | US | 11298485 | B2 | 12 April 2022 |
| | | | | US | 2022313934 | A1 | 06 October 2022 |
| | | | | EP | 3998940 | A1 | 25 May 2022 |
| | | | | EP | 3998940 | A4 | 09 August 2023 |
| | | | | WO | 2021011748 | A1 | 21 January 2021 |
| CN | 108348718 | A | 31 July 2018 | US | 2018317808 | A1 | 08 November 2018 |
| | | | | US | 11191447 | B2 | 07 December 2021 |
| | | | | WO | 2017077417 | A1 | 11 May 2017 |
| | | | | JP | 2018532509 | A | 08 November 2018 |
| | | | | JP | 6831377 | B2 | 17 February 2021 |
| | | | | EP | 3370811 | A1 | 12 September 2018 |
| US | 2009159082 | A1 | 25 June 2009 | GB | 0816825 | D0 | 22 October 2008 |
| | | | | GB | 2455844 | A | 24 June 2009 |

Form PCT/ISA/210 (patent family annex) (July 2022)

**INTERNATIONAL SEARCH REPORT**
Information on patent family members

International application No.

**PCT/CN2023/072158**

| Patent document cited in search report | Publication date (day/month/year) | Patent family member(s) | | | Publication date (day/month/year) |
|---|---|---|---|---|---|
| | | GB | 2455844 | B | 11 July 2012 |
| | | DE | 102007062214 | B3 | 06 August 2009 |
| | | DE | 102007062214 | C5 | 21 December 2017 |
| | | SE | 0802624 | L | 22 June 2009 |
| | | SE | 533075 | C2 | 22 June 2010 |
| | | US | 8109269 | B2 | 07 February 2012 |

Form PCT/ISA/210 (patent family annex) (July 2022)